# EUROPEAN PATENT SPECIFICATION

(11) **EP 3 928 738 B1**
(45) Date of publication and mention of the grant of the patent: **04.09.2024**
(21) Application number: 19916363.5
(22) Date of filing: 17.07.2019
(51) Int. Cl.: A61C 13/00, A61C 13/097, A61C 9/00, A61C 19/05, A61C 19/045, A61C 13/34, A61C 8/00, G06T 19/20

(54) **DENTAL RESTORATION MANUFACTURING METHOD**
VERFAHREN ZUR HERSTELLUNG EINER ZAHNRESTAURATION
PROCÉDÉ DE FABRICATION DE RESTAURATION DENTAIRE

(30) Priority: 21.02.2019 KR 20190020306; 21.02.2019 KR 20190020310; 21.02.2019 KR 20190020314; 21.02.2019 KR 20190020318; 30.05.2019 KR 20190063596
(43) Date of publication of application: 29.12.2021
(73) Proprietor: DIO Corporation, Busan 48058 (KR)
(72) Inventor: CHOI, Byung Ho, Wonju-si Gangwon-do 26434 (KR); JUNG, Seung Mi, Wonju-si Gangwon-do 26418 (KR); KIM, Jin Cheol, Yangsan-si Gyeongsangnam-do 50501 (KR); KIM, Jin Baek, Busan 48107 (KR)
(74) Representative: Beck & Rössig European Patent Attorneys
(86) International application number: PCT/KR2019/008815
(87) International publication number: WO 2020/171314

(56) References cited:
- WO-A1-2011/103876
- WO-A1-2019/004850
- WO-A2-2012/021816
- KR-B1- 101 777 261
- KR-B1- 101 797 150
- KR-B1- 101 857 951
- KR-B1- 101 862 815
- KR-B1- 101 913 586
- US-A1- 2015 305 839

## Description

The present invention relates to a method of manufacturing a dental restoration .

Generally, a dental restoration means oral artificial periodontal tissue which artificially restores an exterior and a function by replacing a destroyed tooth.

In detail, when a natural tooth is lost and left as it is, a distortion occurs in the dentition of adjoining teeth and a correspondingly matching tooth of the destroyed tooth such that deformation of a facial shape may be caused and a masticatory function degraded such as to add discomfort to daily life. In addition, when a loss state of the natural tooth continues for a long time, the alveolar bone surrounding the destroyed tooth is absorbed into the body so that it is difficult to install artificial periodontal tissue.

Here, the dental restoration is installed in the oral cavity to restore the masticatory function and to prevent the correspondingly matching tooth or a residual tooth on a corresponding dental arch side which requires a dental restoration from being deformed or damaged. Here, the dental restoration may be provided as an artificial crown individually matched with the destroyed tooth and may be provided as a shape replacing a plurality of or all destroyed teeth of the corresponding dental arch side. The dental restoration is manufactured using titanium or the like with no adverse reaction in the human body to replace a destroyed dental root and is fixed to an inside of the oral cavity using an implantation material (fixture) to be implanted into the alveolar bone. Accordingly, since there is no secondary cause of tooth decay, the dental restoration may be used stably. Also, since the dental restoration has a structure substantially equal to the natural tooth, gum pain and a feeling of irritation are not present and the dental restoration is semipermanently usable with good care.

Meanwhile, in order to suitably design the dental restoration in a person to be treated and install it at an accurate position, accurate and precise oral information is necessary. To this end, oral surface information and internal tissue information such as an alveolar bone shape, bone density, and/or the like are all required. Accordingly, an image matching process of obtaining a three-dimensional external shape image and a three-dimensional image corresponding to an upper jaw and a lower jaw of a person to be treated and aligning and arranging the same corresponding to an occlusal vertical dimension is necessary.

In detail, the three-dimensional external shape image is obtained by gathering scanned pieces of information using an oral scanner along the oral cavity of the person to be treated. Here, the three-dimensional external shape image is distorted to have a curvature of teeth different from that of a real oral cavity while the scanned pieces of information are gathered. Accordingly, an operation of adjusting the distorted curvature of teeth in the three-dimensional external shape image on the basis of a three-dimensional image obtained through computerized tomography (CT) is necessary.

Also, to prepare an implantation material suitable for a corresponding dental arch side of the person to be treated and to set a height of the dental restoration, the three-dimensional image may be obtained through CT while the upper jaw and the lower jaw are occluded in the vertical dimension of the person to be treated.

However, when any one dental arch of the upper and lower jaws is completely edentulous jaw or partially edentulous jaw in which most teeth are destroyed, it is difficult to space the upper and lower jaws corresponding to the occlusal vertical dimension suitable for the person to be treated. Accordingly, to align and match the three-dimensional external shape image and the three-dimensional image corresponding to the occlusal vertical dimension, a process of precisely calculating the occlusal vertical dimension is necessary.

In detail, general methods of calculating an occlusal vertical dimension of a person to be treated include a method of taking an impression and calculating an occlusal vertical dimension using a dental articulator, a method of using a dental tracer, a method of using a splint customized according to a person to be treated, and the like.

First, in the method of taking the impression and using the dental articulator, a tray is filled with an impression material, an impression of an oral cavity is taken, and an occlusal vertical dimension is calculated while an interval of a plaster model manufactured using the same is adjusted using the dental articulator. The method has a complicated process including taking the impression, manufacturing the plaster model, and the like, and the occlusal vertical dimension is not directly calculated from the oral cavity of the person to be treated, and thus precision thereof is degraded.

Also, in the method of using the dental tracer, a tracing stylus and a tracing plate are installed on an upper jaw and a lower jaw, respectively and an occlusal vertical dimension is calculated while a spaced interval is adjusted in consideration of an interjaw relation. This method has discomfort in that a person to be treated repeatedly opens and closes his/her mouth while a height of the tracing stylus is adjusted to correspond to the occlusal vertical dimension.

Also, in order to precisely install the tracing plate on the upper and lower jaws, it is necessary to manufacture an additional plaster model. That is, after the plaster model is manufactured, positions of the tracing plate and the tracing stylus are aligned using resin, putty, or wax. Also, there is a problem in that excessive time is required for measuring the occlusal vertical dimension such as installing the tracing plate to which the resin, putty, or wax is attached in the oral cavity.

Also, in the method of using the customized splint, an image of an impression model or an oral cavity of a person to be treated is obtained and a splint is separately designed and manufactured on the basis of the image. Subsequently, the manufactured splint is installed in the oral cavity after laminating/placing a wax rim and resin on inner and outer surfaces of the splint, respectively, so as to calculate an occlusal vertical dimension. This method has a problem in that the number of visits to a dental clinic, by the person to be treated, increases to perform an operation of manufacturing the splint, an operation of obtaining an image using the manufactured splint, and the like.

Also, since the probability that an error occurs in a process of transferring the impression model or obtained image to a manufacturer is increased, the reliability of a separately manufactured splint decreases. In addition, since the splint is customized for each person to be treated, there is a problem that an overall cost and period required for tooth restoration increase.

Meanwhile, when the dental restoration is manufactured, in order to manufacture an artificial crown including an occlusal surface substantially shape-matched with a temporomandibular joint of a person to be treated by determining a level jaw position corresponding to an occlusion state of the person to be treated, a gothic arch tracer has been studied and developed.

In detail, conventionally, a tracing plate configured to obtain a gothic arch and a tracking stylus configured to record the gothic arch on the tracking plate are installed outside or inside an oral cavity of the person to be treated and a gothic arch with respect to temporomandibular jaw movement of the person to be treated is obtained.

However, since an operation of providing and installing a gothic arch tracer corresponding to the oral cavity of the person to be treated in the oral cavity is further performed to obtain the gothic arch of the person to be treated, a treatment process was complicated and an excessive time was consumed to obtain the gothic arch.

WO 2019/004850 A1 discloses a method of recording the movement and geometry of the temporomandibular joint. A virtual spatial geometric model of the stomatognathic system is obtained. The geometry of the skull base with the maxilla is selected on the virtual spatial geometric model independently from the geometry of the mandible with condylar heads. On the spatial geometric model, a virtual reference coordinate system and a virtual measurement coordinate system are built. A physical measurement system contains at least one reference sensor that records the values of displacement and/or angular position within the space of the local reference coordinate system as well as at least one measurement sensor that records the values of displacement and/or angular position within the space of the local measurement coordinate system. Anatomical positions of the sensors correspond to at least one measurement and reference point marked on the virtual geometric model. During mandibular movements, signals from measurement and reference sensors are recorded and transferred into the virtual spatial geometric model and then the image of the spatial mandibular volume movement is created. The trajectory of the individual tracing points in the form of curves are generated, between which surfaces are extended, creating a dimensional surface that maps the condylar head functioning within the temporomandibular joint.

WO2023/021816 discloses a method of obtaining a gothic arch motion image.

The present invention is directed to improving precision and reliability in method of manufacturing a dental restoration.

This technical problem is solved by the methods of claims 1 and 9. Advantageous embodiments are indicated in further claims.

According to the embodiment of the present invention, the following effects are provided.

First, as a three-dimensional region of a virtual masticatory surface which is overlapped with a matching target portion is eliminated by a planning part when design information of a dental restoration virtually disposed in a three-dimensional planning image moves along a three-dimensional motion track, the design information of the dental restoration is precisely corrected so as to significantly improve manufacturing precision with respect to a masticatory surface of a finally manufactured dental restoration.

Second, since the virtual masticatory surface is set to be recessed like a gothic arch shape corresponding to a three-dimensional motion track of a temporomandibular joint and eliminated so that a real masticatory surface is manufactured on the basis of the corrected design information of the dental restoration, an additional correction operation of the dental restoration which is needed after being finally manufactured may be minimized so as to significantly improve convenience in treatment.

Third, unlike a conventional case in which an additional tracing plate is mounted in an oral cavity and only a two-dimensional gothic arch is obtained, since a digitalized motion image with respect to the three-dimensional motion track of the temporomandibular joint is quickly obtained using a dynamic scanner while an oral outer surface is exposed, an operation time may be significantly reduced and a reliability of the design information of the dental restoration which is obtained thereby may be significantly improved.

In the Drawings:
FIG. 1 is a flowchart illustrating a dental restoration manufacturing method according to a first embodiment of the present invention.
FIG. 2 is a block diagram illustrating a dental restoration manufacturing system used in the dental restoration manufacturing method according to the first embodiment of the present invention.
FIGS. 3A and 3B are front and plan views in which a part of a wax bite for scanning applied to the dental restoration manufacturing method according to a first embodiment of the present invention is projected.
FIG. 4 is an exemplary view illustrating a first modified example of the wax bite for scanning applied to the dental restoration manufacturing method according to the first embodiment of the present invention.
FIGS. 5A and 5B are exemplary views illustrating a second modified example of the wax bite for scanning applied to the dental restoration manufacturing method according to the first embodiment of the present invention.
FIG. 6 is an exemplary view illustrating a process of obtaining a motion image in the dental restoration manufacturing method according to the first embodiment of the present invention.
FIG. 7 is an exemplary view illustrating a state in which a three-dimensional planning image and the motion image are overlapped in the dental restoration manufacturing method according to the first embodiment of the present invention.
FIG. 8 is an exemplary view illustrating a state in which the three-dimensional planning image and the motion image are eliminated in the dental restoration manufacturing method according to the first embodiment of the present invention.
FIG. 9 is an exemplary cross-sectional view illustrating a process of correcting the wax bite for scanning in the dental restoration manufacturing method according to the first embodiment of the present invention.
FIG. 10 is an exemplary view illustrating a process of obtaining an integrated scanned image in the dental restoration manufacturing method according to the first embodiment of the present invention.
FIGS. 11A and 11B are exemplary views illustrating a process of swapping images in the dental restoration manufacturing method according to the first embodiment of the present invention.
FIG. 12 is a front view illustrating a third modified example of the wax bite for scanning according to the first embodiment of the present invention.
FIG. 13 is a front view illustrating a fourth modified example of the wax bite for scanning according to the first embodiment of the present invention.
FIG. 14 is a front view illustrating a fifth modified example of the wax bite for scanning according to the first embodiment of the present invention.
FIG. 15 is a flowchart illustrating a motion scanning method for manufacturing the dental restoration using the wax bite according to the first embodiment of the present invention.
FIGS. 16A and 16B are front and plan views in which a part of a wax bite for scanning according to a second embodiment of the present invention is projected.
FIG. 17 is a plan view illustrating a modified example of the wax bite for scanning according to the second embodiment of the present invention.
FIG. 18 is an exemplary cross-sectional view illustrating a process of correcting the wax bite for scanning according to the second embodiment of the present invention.
FIG. 19 is an exemplary view illustrating a process of obtaining an integrated scanned image using the wax bite for scanning according to the second embodiment of the present invention.
FIG. 20 is an exemplary view illustrating a process of overlapping and matching a computerized tomography (CT) image obtained using the wax bite for scanning according to the second embodiment of the present invention with the integrated scanned image.
FIG. 21 is a flowchart illustrating a dental restoration manufacturing method according to the second embodiment of the present invention.
FIG. 22 is an exemplary view illustrating a process of designing a temporary dental restoration in the dental restoration manufacturing method according to the second embodiment of the present invention.
FIG. 23 is an exemplary view illustrating a process of correcting the temporary dental restoration in the dental restoration manufacturing method according to the second embodiment of the present invention.
FIG. 24 is an exemplary view illustrating a process of obtaining a motion image in the dental restoration manufacturing method according to the second embodiment of the present invention.
FIG. 25 is an exemplary view illustrating a process of designing a metal frame and an artificial tooth in the dental restoration manufacturing method according to the second embodiment of the present invention.
FIG. 26 is a flowchart illustrating a dental restoration manufacturing method according to a third embodiment of the present invention.
FIG. 27 is an exemplary view illustrating a process of designing a temporary dental restoration in the dental restoration manufacturing method according to the third embodiment of the present invention.
FIG. 28 is an exemplary view illustrating a temporary dental restoration in a dental restoration according to the third embodiment of the present invention.
FIG. 29 is an exemplary view illustrating a process of correcting the temporary dental restoration in the dental restoration manufacturing method according to the third embodiment of the present invention.

Exemplary embodiments of the present invention will be described below in detail with reference to the attached drawings.

Hereinafter, a wax bite for scanning, a motion scanning method for manufacturing a dental restoration using the same, and dental restoration manufacturing method and system according to exemplary embodiments of the present invention will be described in detail with reference to the attached drawings.

Here, the present invention may be applicable to partially edentulous jaw or completely edentulous jaw which has a large amount of tooth loss on at least one side of an upper jaw and a lower jaw such that it is difficult to calculate an occlusal vertical dimension VD.

Also, in the present invention, it may be preferably understood that an implantation target portion means a jaw where a dental restoration is substantially installed and a matching target portion means a jaw occluding with the implantation target portion. Hereafter, it will be described and illustrated that the implantation target portion includes a lower jaw of an oral cavity or an impression model prepared corresponding to the lower jaw, and the matching target portion includes an upper jaw of the oral cavity or an impression model prepared corresponding to the upper jaw. However, in the present invention, the implantation target portion may include an upper jaw or an impression model prepared corresponding to the upper jaw and the matching target portion may include a lower jaw or an impression model prepared corresponding to the lower jaw.

Also, in the present invention, a dental restoration may be preferably understood to include an artificial crown provided to separately correspond to a destroyed tooth and partial/full dentures and partial/full prosthetics including a plurality of such artificial crowns as one set. The dental restoration may be fixed to an oral cavity using a dental implantation material such as a fixture and an abutment. Here, the fixture and the abutment may be prepared as one of ready-made articles or separately manufactured, and the fixture and the abutment may be provided to have one body. Also, the dental restoration may be designed according to a person to be treated in consideration of occlusion with a matching tooth but, on a case-by-case basis, may be standardized to be designed and manufactured on the basis of standard teeth-set data prestored in a planning portion.

In addition, in the present invention, the person to be treated means a patient who needs tooth restoration, and a practitioner and a practitioner side mean a dentist and a dental clinic where an installation of a dental restoration is performed, respectively. Also, a manufacturer and a manufacturer side mean a prosthetist and a prosthetic material manufacturer manufacturing/supplying dental restorations and tools for installing the same.

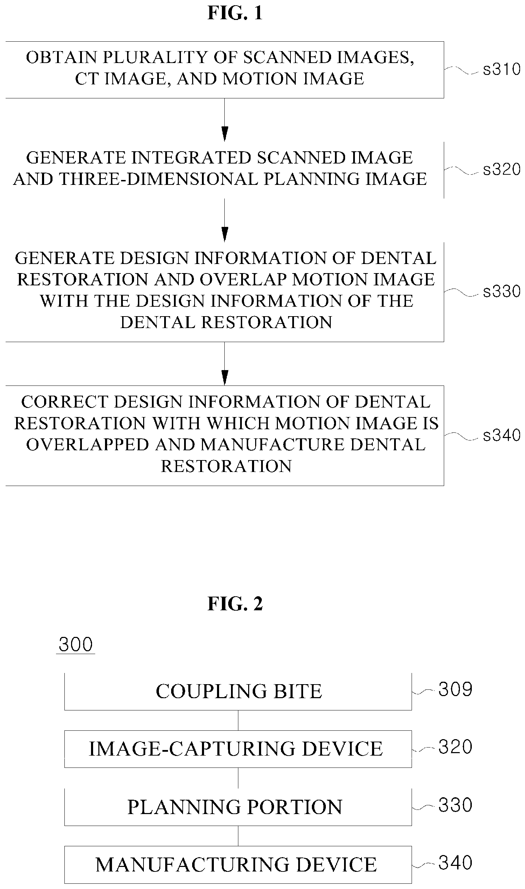

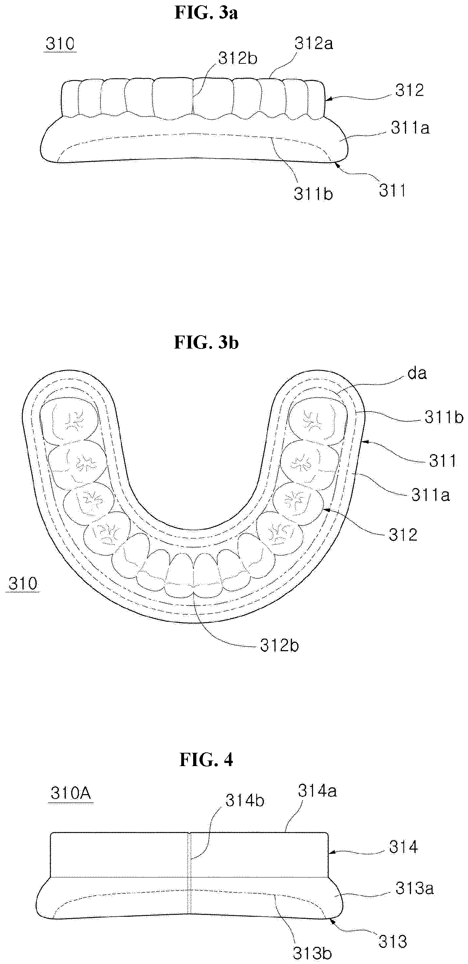

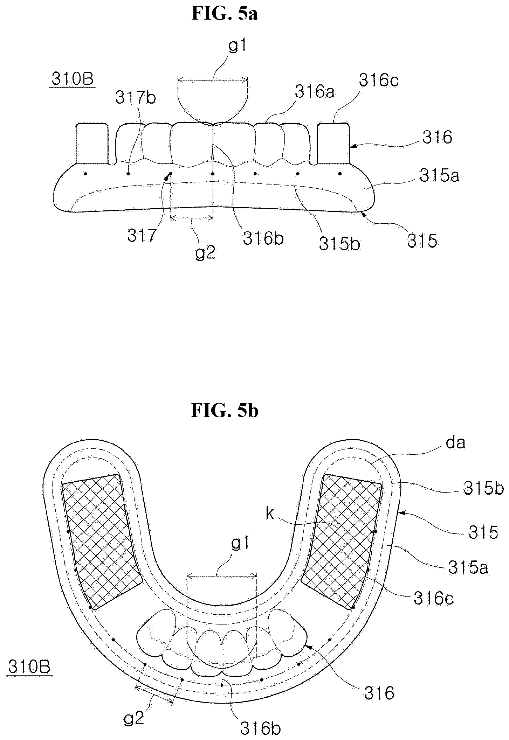

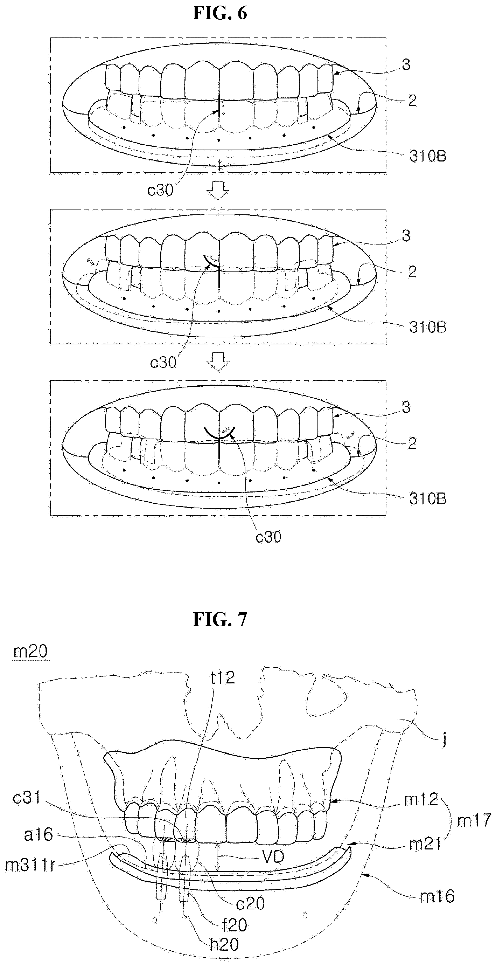

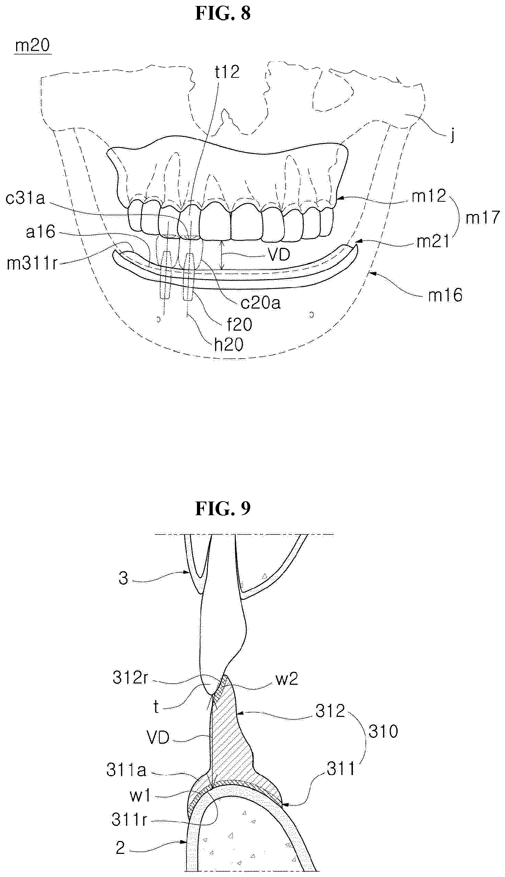

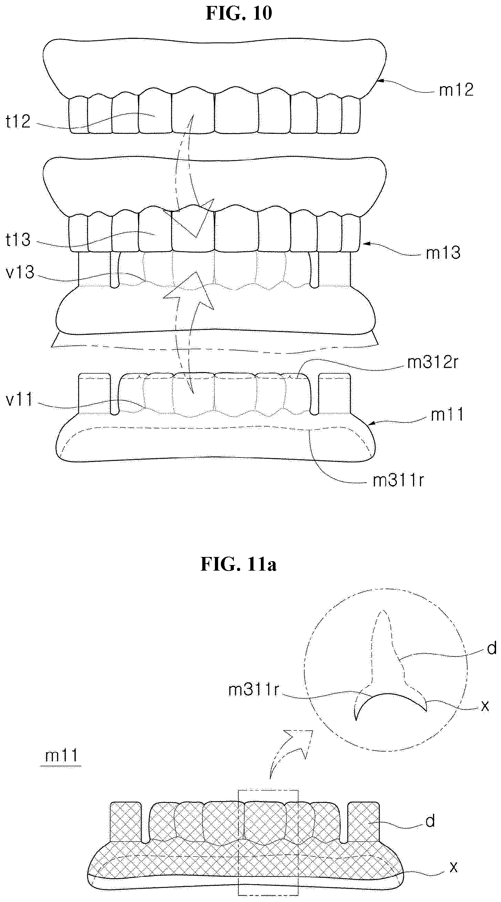

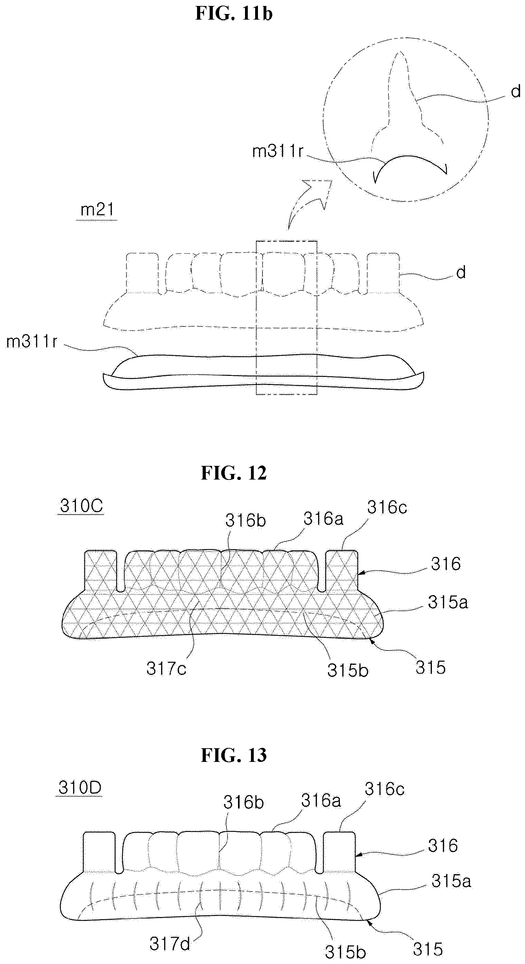

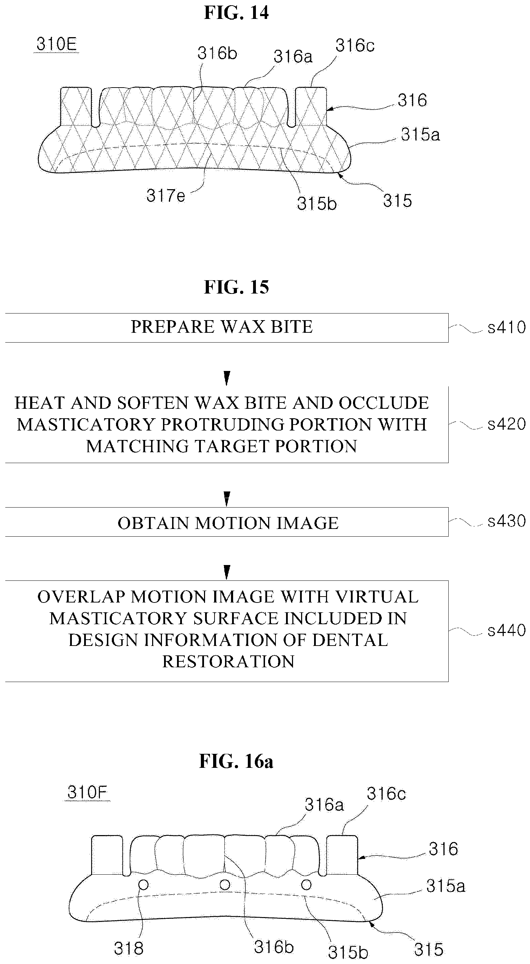

FIG. 1 is a flowchart illustrating a dental restoration manufacturing method according to a first embodiment of the present invention, and FIG. 2 is a block diagram illustrating a dental restoration manufacturing system used in the dental restoration manufacturing method according to the first embodiment of the present invention. Also, FIGS. 3A and 3B are front and plan views in which a part of wax bite for scanning applied to the dental restoration manufacturing method according to a first embodiment of the present invention is projected. Also, FIG. 4 is an exemplary view illustrating a first modified example of the wax bite for scanning applied to the dental restoration manufacturing method according to the first embodiment of the present invention. Also, FIGS. 5A and 5B are exemplary views illustrating a second modified example of the wax bite for scanning applied to the dental restoration manufacturing method according to the first embodiment of the present invention. Also, FIG. 6 is an exemplary view illustrating a process of obtaining a motion image in the dental restoration manufacturing method according to the first embodiment of the present invention. Also, FIG. 7 is an exemplary view illustrating a state in which a three-dimensional planning image and the motion image are overlapped in the dental restoration manufacturing method according to the first embodiment of the present invention. Also, FIG. 8 is an exemplary view illustrating a state in which the three-dimensional planning image and the motion image are eliminated in the dental restoration manufacturing method according to the first embodiment of the present invention. Also, FIG. 9 is an exemplary cross-sectional view illustrating a process of correcting the wax bite for scanning in the dental restoration manufacturing method according to the first embodiment of the present invention. Also, FIG. 10 is an exemplary view illustrating a process of obtaining an integrated scanned image in the dental restoration manufacturing method according to the first embodiment of the present invention. Also, FIGS. 11A and 11B are exemplary views illustrating a process of swapping images in the dental restoration manufacturing method according to the first embodiment of the present invention. Also, FIG. 12 is a front view illustrating a third modified example of the wax bite for scanning according to the first embodiment of the present invention, and FIG. 13 is a front view illustrating a fourth modified example of the wax bite for scanning according to the first embodiment of the present invention. Also, FIG. 14 is a front view illustrating a fifth modified example of the wax bite for scanning according to the first embodiment of the present invention, and FIG. 15 is a flowchart illustrating a motion scanning method for manufacturing the dental restoration using the wax bite according to the first embodiment of the present invention.

As shown in FIGS. 1 to 15, the dental restoration manufacturing method according to the first embodiment of the present invention includes a series of operations of obtaining a plurality of scanned images, a plurality of computerized tomography (CT) images, and a motion image (s310), generating an integrated scanned image and a three-dimensional planning image (s320), generating design information of a dental restoration and overlapping the motion image (s330), and correcting and manufacturing the dental restoration (s340). Also, a motion scanning method for manufacturing a dental restoration using a wax bite according to the first embodiment of the present invention includes a series of operations of preparing a wax bite (s410), heating and softening the wax bite and occluding a masticatory protruding portion with a matching target portion (s420), obtaining a motion image (s430), and overlapping the motion image with a virtual masticating surface included in design information of a dental restoration (s440).

Also, the dental restoration manufacturing method according to the present invention may be performed using a dental restoration manufacturing system 300 including a coupling bite 309, an image capturing device 320, a planning portion 330, and a manufacturing device 340. Here, although the coupling bite 309 is illustrated and described as being provided as a wax bite 310 for scanning in the first embodiment of the present invention as an example, the coupling bite 309 is not limited to the wax bite 310 for scanning.

In detail, the coupling bite 309 may include an inner surface portion shape-matched with an implantation target portion and an outer surface portion correspondingly occluding with a matching target portion. Here, the coupling bite 309 may be provided so that a gap between the corrected inner surface portion and the corrected outer surface portion is a thickness corresponding to an occlusal vertical dimension VD for each person to be treated. Here, the occlusal vertical dimension for each person to be treated may be understood as a gap between upper and lower jaws at which occlusion may be suitably and comfortably performed while the person to be treated occludes the upper and lower jaws. That is, the coupling bite 309 may be understood as a tool to which the implantation target portion and the matching target portion are coupled to correspond to the occlusal vertical dimension VD. Accordingly, respective image data in consideration of the occlusal vertical dimension VD while the implantation target portion and the matching target portion occlude with each other due to the coupling bite 309 may be obtained.

Here, corresponding to the occlusal vertical dimension for each person to be treated may be understood as including premanufacturing the gap between the inner surface portion and the outer surface portion of the coupling bite 309 to correspond to the occlusal vertical dimension for each person to be treated and installing the coupling bite 309 between the implantation target portion and the matching target portion while being corrected to correspond to the occlusal vertical dimension for each person to be treated due to an occlusion pressure. Hereinafter, it will be described and illustrated as an example that the coupling bite 210 is provided as the wax bite 310 for scanning.

Here, the wax bite 310 for scanning is a tool disposed between the implantation target portion and the matching target portion so as to align and match respective pieces of image data obtained to design the dental restoration in consideration of an occlusal vertical dimension of a person to be treated.

Here, the wax bite 310 for scanning is provided as one of ready-made articles and may be formed of a material that is correctable so as to correspond to an occlusal vertical dimension suitable for each person to be treated. Accordingly, in a process of obtaining each piece of image data required in designing the dental restoration, a precise occlusal vertical dimension may be guided through the wax bite 310 for scanning corrected according to the person to be treated. Here, each piece of the image data may be understood as a concept including a plurality of such scanned images and the CT image.

Also, the image capturing device 320 may be understood as including an oral scanner, a CT apparatus, and a dynamic scanner. In detail, the plurality of scanned images, which include three-dimensional surface information on outer surfaces of the implantation target portion, the matching target portion, and the wax bite 310 for scanning, are obtained using the oral scanner. Also, the CT image that includes internal tissue information on densities, alveolar bone shapes, and the like of the upper and lower jaws is obtained using the CT apparatus. In addition, a gothic arch motion image of a three-dimensional motion track with respect to temporomandibular joint movement in a left and right direction, a front-rear direction, and a vertical direction is obtained using the dynamic scanner while an outer surface of the implantation target portion is exposed and a masticatory protruding portion and the matching target portion occlude with each other.

Also, the planning portion 330 may be understood as a design device configured to design the dental restoration on the basis of respective pieces of image data which are obtained using the image capturing device 320 and prestored design information. In addition, an implantation plan for a dental implantation material such as a fixture and an abutment for fixing the dental restoration in the oral cavity may be devised through the planning portion 330. Also, tooth restoration through implantation of a dental implantation material and a dental restoration may virtually be simulated on the basis of the devised implantation plan. In addition, the dental restoration is designed using the planning portion 330 while the motion image may be overlapped with the three-dimensional planning image so as to display and eliminate an overlapped region to be eliminated in an overlapped occluding region in a virtual masticating surface.

Meanwhile, the manufacturing device 340 may be understood as a three-dimensional printer, a milling device, or the like configured to manufacture real articles by three-dimensional printing or milling to correspond to the generated dental restoration through the planning portion 330 and design information of the surgical guide. However, the manufacturing device 340 may further include a molding device provided to correspond to designed information.

Meanwhile, referring to FIGS. 3A to 5B, the wax bite 310, 310A, or 310B disposed between the implantation target portion and the matching target portion is provided. Here, the wax bite 310, 310A or 310B may be formed to be standardized corresponding to a preset standard dental arch profile da, and a variable shape-matching portion 311, 313, or 315 and a masticatory protruding portion 312, 314, or 316 may be integrally formed. Here, the wax bites 310, 310A, or 310B for scanning may be provided to obtain the occlusal vertical dimension of the person to be treated and to obtain the motion image.

In detail, the wax bite 310, 310A, or 310B for scanning may have an overall arch shape in which an outer surface portion 312a, 314a, or 316a protrudes from one matching side between the implantation target portion and the matching target portion while an inner surface portion 311b, 313b, or 315b is recessed to a certain depth. The arch shape may correspond to the standard dental arch profile da. Here, the standard dental arch profile da may be understood as a surface having a certain area corresponding to an interval between an outside and an inside of a gingival portion surrounding the alveolar bone that is a part of the implantation target portion where teeth are substantially arranged. Here, the outside of the gingival portion may be understood as a part corresponding to a labial side and a buccal side, and the inside of the gingival portion may be understood as a part corresponding to a lingual side.

The standard dental arch profile da may be standardized and calculated in consideration of anatomical deviations by age and gender. In detail, the standard dental arch profile da may be calculated while being classified stage by stage in response to an average value representing an arch size by age and gender. Accordingly, the wax bite 310, 310A, or 310B for scanning may be provided for general use while being classified to have preset sizes. For example, the wax bite 310, 310A, or 310B for scanning may be provided while being provided as generally standardized ready-made article sets classified into six sides, that is, large/medium/small of an upper jaw side and large/medium/small of a lower jaw side.

That is, since the occlusal vertical dimension is guided using the wax bite 310, 310A, or 310B for scanning mass-produced as ready-made articles in the present invention, a period of time consumed for calculating the occlusal vertical dimension may be reduced and an overall cost may be economically reduced while the dental restoration is designed. Accordingly, a general problem of excessive time and cost being consumed for calculating the occlusal vertical dimension using a tracer or a customized splint may be remedied.

In detail, since the tracer needs an additional impression model to be accurately installed in the oral cavity and a technician who substantially adjusts a tracing stylus is a practitioner or a manufacturer, a difference from an occlusal vertical dimension determined by a person to be treated may occur. Also, in the case of a separately customized splint, inaccurate information may be reflected due to an error in a process of repeatedly transmitting/transferring information for designing the splint between the practitioner side and the manufacturer side. On the other hand, in the present invention, since a practitioner who determines mastication sensitivity and corrects a thickness and a shape of the wax bite 310, 310A, or 310B for scanning is actually the person to be treated on the basis thereof, an occlusal vertical dimension optimized for each person to be treated may be reflected. Accordingly, precision and accuracy of a dental restoration designed and manufactured on the basis of the accurately calculated occlusal vertical dimension may be significantly improved.

In addition, the wax bite 310, 310A, or 310B for scanning may be a provided as a ready-made article and may be mass-produced while being standardized in a plurality of sizes to be generally applicable to oral cavities of a variety of people to be treated or impression models corresponding thereto. Accordingly, since the practitioner side provides the wax bite 310, 310A, or 310B for scanning by size in advance to be instantaneously usable when the person to be treated visits, convenience in use may be significantly improved. Also, the number of visits to a dental clinic by the person to be treated may be substantially minimized to one time for an operation of obtaining information on the implantation target portion and the matching target portion before installing the dental restoration.

Also, the plurality of scanned images, the CT image, and the motion image may be obtained as soon as the wax bite 310, 310A, or 310B for scanning is corrected to be suitable for each person to be treated. Here, since the time points of obtaining the plurality of scanned images, the CT image, and the motion image are substantially equal, a matching rate of respective pieces of image data may be significantly improved.

Meanwhile, the variable shape-matching portion 311, 313, or 315 is provided on an inner surface portion side of the wax bite 310, 310A, or 310B for scanning and may be formed to have a margin 311a, 313a, or 315a which exceeds a width of a standard gingival portion corresponding to the implantation target portion. Also, the masticatory protruding portion 312, 314, and 316 may be provided on an outer surface portion side of the wax bite 310, 310A, or 310B for scanning and may be integrally formed with the variable shape-matching portion 311, 313, and 315 while protruding to correspond to the matching target portion.

In addition, referring to FIGS. 3A and 3B, the masticatory protruding portion 312 may be formed to have an arch shape corresponding to the preset standard dental arch profile da while having an outer surface corresponding to an exterior of a tooth. That is, the masticatory protruding portion 312 may be formed to include a masticating surface corresponding to a real tooth shape and an interdentium corresponding portion 312b.

Here, the interdentium corresponding portion 312b may be shown as engraving and may be used as an alignment marker that becomes an alignment reference for disposition between the implantation target portion and the matching target portion. In detail, the alignment marker may be aligned corresponding to a preset visible reference marker portion on any one side of the implantation target portion and the matching target portion. Here, the visible reference marker portion may be formed as an interdentium between residual teeth on a correspondingly matching side, and in some cases, the maxillary labial frenum or the mandibular labial frenum corresponding to the midline of the human body may be set as the visible reference marker.

Accordingly, when the wax bite 310 for scanning is corrected, inner and outer surface portions may be accurately corrected corresponding to the implantation target portion and the correspondingly matching portion. Also, even when the corrected wax bite 310 for scanning is repeatedly installed/separated between and from the implantation target portion and the matching target portion, an accurate position may be uniformly guided.

Alternatively, as shown in FIG. 4, the masticatory protruding portion 314 may have a simply protruding shape with a cross section corresponding to the preset standard dental arch profile. In this case, a shape of the wax bite 310 for scanning is simplified and easily manufactured so that productivity may be improved and manufacturing costs may be reduced. Here, the alignment marker 314b may be further included in the masticatory protruding portion 314 and a preset outer surface of the variable shape-matching portion 313 integrally formed therewith. The alignment marker 314b may be manufactured in the displayed state when the wax bite 310 for scanning is mass-produced and, in some cases, may be displayed by a practitioner using a tool in an operation of correcting the wax bite 310 for scanning.

Also, as shown in FIGS. 5A and 5B, in the masticatory protruding portion 316, a boundary area between an anterior tooth portion and a molar tooth portion is recessed toward the variable shape-matching portion 315 so as to divide the anterior tooth portion and the molar tooth portion. Here, an outer surface 316a of the masticatory protruding portion on an anterior tooth portion side may be formed to be an outer surface corresponding to an exterior of a tooth. That is, the masticatory protruding portion 316 may be formed to include a masticating surface corresponding to a real tooth shape and an interdentium corresponding portion 316b.

Also, an outer surface 316c of the masticatory protruding portion on a molar tooth portion side may be divided from the outer surface 316a of the masticatory protruding portion on an anterior tooth portion side and may protrude with a cross-sectional area corresponding to the preset standard dental arch profile. Here, a friction marker k including at least any one of a scratch and intaglio may be formed on an occlusal surface of the masticatory protruding portion 316 to improve adhesion with a curable resin filled when relining to correspond to the occlusal vertical dimension of the person to be treated. Here, the friction marker k may be formed as at least any one of a plurality of scratches and a plurality of intaglios so as to increase a surface area of the masticatory protruding portion 316.

Here, the masticatory protruding portion 316 and the matching target portion may be filled with an additional dental resin for occlusal vertical dimension alignment therebetween and occlude with each other so as to be relined. The dental resin may be provided as alginate, putty, a curable resin, or the like which is generally used when an impression is obtained in a dental clinic.

Accordingly, a surface area for adhesion between the occlusal surface of the masticatory protruding portion 316 and the curable resin increases corresponding to the masticatory protruding portion 316 including the friction marker k scratched or engraved in the occlusal surface and the occlusal vertical dimension of the person to be treated when the occlusal surface of the masticatory protruding portion 316 is filled with the curable resin. Accordingly, the adhesion between the masticatory protruding portion 316 and the curable resin may improve so as to obtain a scanned image with a precisely calculated occlusal vertical dimension.

Also, the masticatory protruding portion 316 may include a recessed portion in which the boundary area between the anterior tooth portion and the molar tooth portion is recessed toward the variable shape-matching portion 315. Here, the recessed portion may be preferably understood as including a recessed area corresponding to a canine tooth or premolar tooth. Accordingly, the anterior tooth portion formed to be similar to real teeth and the molar tooth portion formed to have a surface area that is increased when relined may be divided from each other on the basis of the recessed portion so as to improve accuracy in obtaining the occlusal vertical dimension and motion image.

In detail, motion image c30 is obtained by motion-scanning a three-dimensional motion track of the temporomandibular joint which moves using the dynamic scanner while the outer surface of the oral cavity is exposed and the masticatory protruding portion 316 and the matching target portion 3 occlude with each other. Here, the three-dimensional motion track is shown as a gothic arch shaped track according to movement of the temporomandibular joint in a three-dimensional space including a left-right direction, a front-rear direction, and a vertical direction on the basis of a reference point such as the interdentium corresponding portion 316b. Here, the motion image c30 may be obtained by motion-scanning based on a reference point such as an upper end point or the like of the interdentium corresponding portion 316b of the wax bite 310B for scanning disposed on the lower jaw. Alternatively, in some cases, when the upper jaw is the implantation target portion and the lower jaw is the matching target portion, motion scanning may be performed on the basis of an interdentium upper end point of a residual tooth on the anterior tooth side of the matching target portion.

Accordingly, unlike a conventional case in which an additional tracking plate is mounted in an oral cavity and only a two-dimensional gothic arch is obtained, the digitalized motion image c30 with respect to the three-dimensional motion track of the temporomandibular joint which is moving is quickly obtained using the dynamic scanner while the outer surface of the oral cavity is exposed. Accordingly, a working time may be significantly reduced and convenience in treatment may be significantly improved so as to significantly improve a reliability of obtained dental restoration design information. In addition, a masticating surface of a finally manufactured artificial crown may be precisely designed corresponding to movement of a temporomandibular joint of each person to be treated. That is, since the motion image c30 obtained using the dynamic scanner includes the gothic arch image with respect to the three-dimensional motion track of the temporomandibular joint, it is possible to obtain a precise gothic arch image unlike a conventional gothic arch tracer.

Here, when the wax bite 310B for scanning is motion-scanned, there is a worry that a motion track of the temporomandibular joint which is moving is not recognized by the dynamic scanner due to being the same color and having a smooth surface structure. That is, when the dynamic scanner scans the movement of the temporomandibular joint, a dynamic image obtained by scanning a motion change of a recognition degradation surface of the wax bite for scanning is converted into a three-dimensional modeling file. Here, a recognition rate of the dynamic scanner is degraded due to the smooth recognition degradation surface during an image conversion process. To redeem this, a motion recognition augmentation portion 317 may be provided/formed on the recognition degradation surface of the wax bite 310B for scanning.

In detail, as shown in FIGS. 5A to 6, the wax bite 310B for scanning may further include the motion recognition augmentation portion 317 disposed on the recognition degradation surface to provide a visible reference marker when a gothic arch motion image c30 with respect to a three-dimensional motion track of the temporomandibular joint j (refer to FIG. 7) of the person to be treated is obtained. Here, the recognition degradation surface means a smooth surface portion of a front surface portion of the wax bite 310B for scanning.

Here, the motion recognition augmentation portion 317 according to the first embodiment of the present invention may be provided as a plurality of matching markers 317b attached along an outer surface of the wax bite 310B for scanning. Here, a plurality of such matching markers may be provided/formed of a radiopaque material and to be spaced apart at three or more places along the outer surface of the wax bite 310B for scanning. The matching markers may be provided as a solid body having a preset volume and attached to the implantation target portion. A dental resin having a density through which radiation does not pass may be injected to a certain size onto the outer surface of the implantation target portion and cured to be attached.

Also, gaps g2 between the respective matching markers 317b may be disposed at intervals that are less than a maximum movement radius g1 of the temporomandibular joint moved in a left-right direction, a front-rear direction, and a vertical direction. Here, when the gap g2 between the matching markers 317b exceeds the maximum movement radius g1 of the temporomandibular joint, a motion track may not be recognized due to being the same color and the wax bite 310B for scanning or the implantation target portion having a smooth surface structure while the temporomandibular joint is motion-scanned by the dynamic scanner.

Accordingly, since a precise motion image is obtained in motion-scanning using the dynamic scanner as the gaps g2 between the matching markers 317b are arranged at intervals that are less than the maximum movement radius g1 of the temporomandibular joint, precision of a finally-manufactured artificial dental crown may be significantly improved.

Meanwhile, referring to FIG. 12, the motion recognition augmentation portion may be provided as a powder spray which is applied to a surface of the wax bite for scanning and forms a discontinuous uneven surface 317c. Here, as the powder spray is applied to the surface of the wax bite for scanning instead of the above matching marker, the surface of the wax bite may be formed as the discontinuous uneven surface 317c. Accordingly, when the temporomandibular joint is motion-scanned by the dynamic scanner, each of such discontinuous uneven surfaces 317c of the surface of the wax bite may provide a reference point with respect to the motion.

Also, referring to FIG. 13, the motion recognition augmentation portion may be marked with a plurality of intaglios 317d at preset intervals along the surface of the wax bite for scanning. In addition, referring to FIG. 14, the motion recognition augmentation portion may be marked with a plurality of places of pattern-laminated insoluble ink 317e on the surface of the wax bite for scanning. Here, when the motion recognition augmentation portion is provided using matching markers, intaglios, and patterns laminated using insoluble ink, gaps between the respective motion recognition augmentation portions may be arranged at intervals that are less than the maximum movement radius of the temporomandibular joint moved in a left-right direction, a front-rear direction, and a vertical direction.

Accordingly, a motion of the motion recognition augmentation portion 317 according to movement of the temporomandibular joint is imaged and displayed in the motion image. Also, data with respect to a three-dimensional motion track of the temporomandibular joint may be obtained, by the planning portion, on the basis of three-dimensional surface information/data of the motion recognition augmentation portion 317. Accordingly, precision of the motion image may be significantly improved.

Also, the motion recognition augmentation portion configured to augment motion recognition is provided on the smooth surface of the wax bite for scanning which is integrally formed using a paraffin wax material. Accordingly, a recognition rate with respect to the three-dimensional motion track in motion scanning using the dynamic scanner may be significantly augmented. In addition, a recognition degradation surface of the wax bite for scanning is augmented by the motion recognition augmentation portion so that the motion image with respect to a three-dimensional motion track of the temporomandibular joint corresponding to a gothic arch shape is accurately obtained as three-dimensional data and reflected on design information of the dental restoration. Accordingly, precision of a finally-manufactured dental restoration may be significantly improved.

Meanwhile, referring to FIGS. 3A to 5B, the margin 311a, 313a, or 315a may be formed to exceed a width of the standard gingival portion. Here, the width of the standard gingival portion may be understood as a standard lateral interval between an outside and an inside of the gingival portion which is calculated in consideration of anatomical deviations by age and gender. The width may be understood as a standardized lateral interval between a labial side and a lingual side of the gingival portion which is calculated in consideration of the anatomical deviations by age and gender. In addition, the width of the standard gingival portion may be understood as a width substantially corresponding to or set to be greater than the standard dental arch profile da.

Accordingly, the margin 311a, 313a, or 315a may be formed to have a laterally extending area rather than the width of the standard gingival portion. Accordingly, the wax bite 310, 310A, or 310B for scanning may be disposed to entirely surround the inside and outside of the gingival portion of the implantation target portion. Also, an inner surface 311b, 313b, or 315b of the variable shape-matching portion 311, 313, or 315 may be formed to be concave with a curvature smaller than that of a convex outer surface profile of the gingival portion of the average implantation target portion side. Accordingly, a suitable marginal volume in which the outer surface of the implantation target portion is corrected to be shape-matched may be secured while coming into overall contact with the variable shape-matching portion 311.

Meanwhile, in the present invention, since an inner surface portion 311b, 313b, or 315b of the wax bite 310, 310A or 310B for scanning is substantially the same as the inner surface 311b, 313b, or 315b of the variable shape-matching portion 311, 313, or 315, it may be understood why they are described and illustrated with the same reference numeral. Also, since an outer surface portion 312a, 314a, or 316a of the wax bite 310, 310A, or 310B for scanning is substantially the same as the outer surface 312a, 314a, or 316a of the masticatory protruding portion 312, 314, or 316, it may be understood why they are described and illustrated with the same reference numeral.

Also, in the wax bite 310, 310A, or 310B for scanning, a thickness between the inner surface 311b, 313b, or 315b of the variable shape-matching portion 311, 313, or 315 and the outer surface 312a, 314a, or 316a of the masticatory protruding portion 312, 314, or 316 may be formed to be greater than an average vertical dimension calculated in consideration of the anatomical deviations by age and gender. Accordingly, while the wax bite 310, 310A, or 310B for scanning which is softened by heating the inner and outer surface portions is disposed between the implantation target portion and the correspondingly matching portion, a suitable spare thickness correctable by a simple occlusion pressure may be secured.

In addition, a reinforcing surface portion (not shown) may be further formed to support a space between both ends of the variable shape-matching portion 311, 313, or 315 that is formed to have an arch shape and the masticatory protruding portion 312, 314, or 316. Accordingly, even when the occlusal pressure is applied while the inner and outer surface portions of the wax bite 310, 310A, or 310B for scanning are softened so that supporting strength is reduced, distortion or fracture may be prevented. Also, in a case of corresponding to the upper jaw side, since occlusion is corrected while the reinforcing surface portion (not shown) is supported by a hard palate, the wax bite 310, 310A, or 310B for scanning may be prevented from sliding and shifting in position in occlusion. Accordingly, the corrected wax bite 310, 310A, or 310B for scanning may be precisely corrected to accurately guide the occlusal vertical dimension.

Meanwhile, referring to FIG. 9, the wax bite 310 for scanning is provided while being standardized as a ready-made article for general use but is selected and used as a product having a size suitable for a person to be treated. Also, the wax bite 310 for scanning is disposed between the implantation target portion 2 and the matching target portion 3 while the variable shape-matching portion 311 facing/corresponding to the implantation target portion 2 is softened when heated to a preset temperature or higher. Also, the masticatory protruding portion 312 facing/corresponding to the matching target portion 3 is softened when heated to a preset temperature or higher. Here, parts w1 and w2 shown in FIG. 9 may be understood as parts softened by heating.

Also, before or after each piece of the image data is obtained, the wax bite 310 for scanning is disposed between the implantation target portion 2 and the matching target portion 3 while the inner and outer surface portions thereof are softened. Here, the inner and outer surface portions of the wax bite 310 for scanning are heated and softened to have lower strength than the implantation target portion 2 and the matching target portion 3. Accordingly, an inner surface profile of the variable shape-matching portion 311 and an outer surface profile of the masticatory protruding portion 312 are deformed to correspond to outer surface profiles of the implantation target portion 2 and the matching target portion 3, respectively, by occlusal pressure. Additionally, a thickness between the variable shape-matching portion 311 and the masticatory protruding portion 312 is deformed to correspond to the occlusal vertical dimension VD.

In detail, the inner surface of the variable shape-matching portion 311 is recessed concavely by the occlusal pressure to correspondingly shape-match with the convex outer surface of the implantation target portion 2. Accordingly, an intaglio groove corresponding to the implantation target portion 2 may be formed in the variable shape-matching portion 311 and corrected as a shape-matching correction portion 311r. Also, the outer surface of the masticatory protruding portion 312 may include a chewing trace formed by the occlusal pressure to be correspondingly occluded with a profile of an end of the matching target portion 3, for example, an end of a matching tooth t, and may be corrected as an occlusal correction portion 312r. In addition, an interval between the shape-matching correction portion 311r and the occlusal correction portion 312r may be corrected to substantially correspond to the occlusal vertical dimension VD.

Also, each piece of the image data obtained while the wax bite 310 for scanning with the shape-matching correction portion 311r and the occlusal correction portion 312r being formed and cured again is installed includes information of the occlusal vertical dimension VD. As described above, since the wax bite 310 for scanning is standardized to have a certain shape and mass-produced, manufacturing time and costs are economically reduced and the wax bite 310 for scanning may be easily corrected to become a surface profile suitable for each person to be treated. Accordingly, precision/accuracy of the occlusal vertical dimension VD and each piece of the image data which is obtained by calculated/obtained using the corrected wax bite 310 for scanning may be significantly improved.

The wax bite 310 for scanning may be formed of paraffin wax. The paraffin wax has a certain supporting strength of a solid at a room temperature and is easily cut and corrected using a tool such as a knife or the like. Accordingly, when a natural/restored tooth remains in the implantation target portion 2 or a length of the wax bite 310 for scanning is long, the paraffin wax may be used after being cut or divided. In addition, when the interdentium corresponding portion 312b (refer to FIG. 3A) or the alignment marker 314b (refer to FIG. 4) is formed on the masticatory protruding portion 312, the paraffin wax may be cut or divided on the basis thereof so that use convenience may be further improved.

Also, the paraffin wax has a melting point within a range of 47 to 64 °C and may have a property of being softened even when high-temperature heat is not applied. Accordingly, the paraffin wax is safe even when being directly applied to the oral cavity when heated and is quickly cured at a temperature less than the preset temperature so that the degree of shape-matching between the implantation target portion 2 and the matching target portion 3 may be maintained. In addition, since the paraffin wax has a low contraction rate during a softening and curing process, the degree of shape-matching rate of the implantation target portion and the matching target portion and precision of the calculated occlusal vertical dimension may be significantly improved.

Here, the paraffin wax includes paraffin (for example, ceresin) at 70 to 85 weight% and other additives at a residual weight% with respect to an entire weight%. For example, the paraffin wax may include ceresin at 77 to 85 weight%, beeswax at 7 to 13 weight%, carnauba wax at 1.5 to 3 weight%, resin at 2 to 4 weight%, and microcrystal wax at 1.5 to 3 weight%.

Meanwhile, the inner surface portion and the outer surface portion of the wax bite 310 for scanning may be heated to a temperature within a range of 40 to 55 °C. Also, the wax bite 310 for scanning which is softened by heating each of the inner and outer surface portions is disposed between the implantation target portion 2 and the matching target portion 3 and occlusal pressure is applied to correspond to a preset occlusal vertical dimension VD. Accordingly, the inner surface portion of the wax bite 310 for scanning is pressurized and corrected to correspond to the outer surface profile of the implantation target portion 2 and is integrally formed with the shape-matching correction portion 311r. Also, the outer surface portion of the wax bite 310 for scanning is corrected to be correspondingly occluded with the matching target portion 3 and is integrally formed with the occlusal correction portion 312r. Here, the interval between the shape-matching correction portion 311r and the occlusal correction portion 312r may be formed to correspond to the occlusal vertical dimension VD.

Here, when the inner surface portion and the outer surface portions of the wax bite 310 for scanning are heated to a temperature less than 40 °C, the wax bite 310 for scanning cannot be softened to have strength lower than the implantation target portion 2 and the matching target portion 3. Accordingly, even when occlusal pressure is applied, it is difficult to correct the inner and outer surface portions of the wax bite 310 for scanning to correspond to the outer surface profiles of the implantation target portion 2 and the matching target portion 3. On the other hand, when the inner and outer surface portions are heated at a temperature higher than 55 °C, the inner and outer surface portions change in phase into liquid excessively. Accordingly, fluidity of the inner and outer surface portions of the wax bite 310 for scanning excessively increases so as not be corrected to have definite shapes corresponding to the implantation target portion 2 and the matching target portion 3. Also, when the wax bite 310 for scanning is directly installed in the oral cavity, there is a risk such as a burn caused by a high temperature and the like.

Accordingly, the inner surface portion and the outer surface portion of the wax bite 310 for scanning may be heated to a temperature within a range of 40 to 55 °C to be softened to have strength lower than the implantation target portion and the matching target portion while maintaining a substantially corrected shape.

In addition, the wax bite 310 for scanning may be heated only at the inner surface portion and the outer surface portion corresponding to the implantation target portion 2 and the matching target portion 3. Accordingly, an overall shape of the wax bite 310 for scanning may be maintained while the inner surface portion and the outer surface portion may be easily corrected to be shape-matched/occluded with the implantation target portion 2 and the matching target portion 3.

Here, the wax bite 310 for scanning may be placed into a container which accommodates warm water at a temperature of 45 to 55 °C to be heated. Also, the wax bite 310 for scanning may be heated using a heating device which discharges hot air, and any means configured to heat and soften the inner and outer surface portions of the wax bite 310 for scanning may be applied to the present invention.

In addition, in order to more precisely form the shape-matching correction portion 311r, the shape-matching correction portion 311r and the implantation target portion 2 may be filled with an additional dental resin therebetween to occlude and be relined. That is, a gap or pore that may occur between the shape-matching correction portion 311r and the implantation target portion 2 as the paraffin wax is cured is filled with the dental resin and cured so that a shape matching rate between the shape-matching correction portion 311r and the implantation target portion 2 may be further improved. The dental resin may include alginate, putty, a curable resin, or the like which are generally used when an impression is taken in a dental clinic and may have a coefficient of viscosity not allowing soft tissue of a gingival portion g of the implantation target portion 2 to be excessively pressurized and deformed.

Meanwhile, the wax bite 310 for scanning may have the inner surface portion corresponding to the implantation target portion 2 and the outer surface portion corresponding to the matching target portion 3 which are integrally formed to have substantially a single body. Accordingly, a different-type of wax and curable resin are laminated on the inner and outer surface portions of an occlusion-checking means such as a tray or a splint so that it is possible to basically remedy a conventional problem that a lamination part slides and is distorted laterally or a position is deformed. That is, since the occlusal pressure is substantially applicable in a vertical direction of the wax bite 310 for scanning, the precision of the calculated occlusal vertical dimension VD may be significantly improved. As described above, on the basis of the wax bite 310 for scanning corrected, the implantation target portion 2 and the matching target portion 3 may be occlusion-arranged to correspond to the occlusal vertical dimension VD optimized for a person to be treated.

Here, when the wax bite 310 for scanning is directly installed in the oral cavity and corrected, the occlusal vertical dimension VD may be determined by an expression of an opinion regarding the direct masticatory sensitivity of the person to be treated or may be determined by measuring electrical/chemical signals of a temporomandibular joint periphery and jaw muscles. That is, as the person to be treated actually occludes upper and lower jaws with each other while the wax bite 310 for scanning is inserted into the oral cavity, the wax bite 310 for scanning may be corrected to correspond to the occlusal vertical dimension VD.

Alternatively, when the implantation target portion and the matching target portion are provided as impression models, the wax bite 310 for scanning may be disposed between the impression models connected to the upper and lower jaw sides through an articulator. Also, as the impression models are occlusion-arranged to correspond to the occlusal vertical dimension VD statistically calculated in advance using the articulator, occlusal pressure may be applied. Accordingly, the inner surface portion and the outer surface portion of the wax bite 310 for scanning may be corrected to be the shape-matching correction portion 311r and the occlusal correction portion 312r.

As described above, in the present invention, while costs are reduced using the wax bite 310 for scanning provided as a ready-made article, surface profiles of the inner surface portion and the outer surface portion thereof and an interval between the surface profiles may be separately corrected according to the occlusal vertical dimension VD of each person to be treated. Accordingly, the occlusal vertical dimension VD optimized for each person to be treated may be precisely calculated so that overall accuracy and precision of tooth restoration may be significantly improved.

Here, the wax bite 310 for scanning may be disposed between the implantation target portion 2 and the matching target portion 3 and corrected according to the occlusal pressure before obtaining each piece of the image data. Alternatively, image data regarding the implantation target portion 2 and the matching target portion 3 before installing the wax bite 310 for scanning may be obtained in advance, and image data regarding the implantation target portion 2 and the matching target portion 3 after installing the wax bite 310 for scanning may be additionally obtained.

Meanwhile, a plurality of scanned images of the implantation target portion and the matching target portion are obtained using an intraoral scanner. Also, a CT image of the oral cavity occluded through the wax bite for scanning is obtained using the CT apparatus. Also, while the implantation target portion and the masticatory protruding portion provided on the coupling bite occlude with each other, a gothic arch motion image of a three-dimensional motion track of the person to be treated is obtained using the dynamic scanner (s310 in FIG. 1).

Here, the plurality of scanned images may include scanned images obtained with respect to the implantation target portion and the matching target portion before the wax bite for scanning is installed. In addition, a plurality of scanned images obtained with respect to the implantation target portion and the matching target portion after the corrected wax bite for scanning is installed may be included.

In detail, referring to FIG. 10, the plurality of scanned images may include a first scanned image m11 of an overall outer surface of the wax bite for scanning corrected so that the shape-matching correction portion and the occlusal correction portion are integrally formed. Also, the plurality of scanned images may include a second scanned image m12 of an outer surface of the matching target portion. Also, a third scanned image m13 of outer surfaces of the implantation target portion and the matching target portion which are occluded with each other through the corrected wax bite for scanning may be included.

Here, in the first scanned image m11, three-dimensional surface information of an overall outer surface of the wax bite for scanning which includes three-dimensional surface information m311r of the shape-matching correction portion and three-dimensional surface information m312r of the occlusal correction portion are shown as an image.

Also, in the second scanned image m12, three-dimensional surface information on the outer surface of the matching target portion is shown as an image. Here, when a matching tooth remains on the matching target portion, three-dimensional surface information t12 on the matching tooth is shown as an image in the second scanned image m12. Alternatively, when the matching target portion side is an edentulous jaw, three-dimensional surface information of the gingival portion of the matching target portion side may be shown as an image in the second scanned image m12. Also, in the operation of generating the three-dimensional planning image, standard teeth-set data of the matching target portion side prestored in the planning portion may be imaged and virtually arranged.

Also, in the third scanned image m13, three-dimensional surface information on an outer surface in an occlusion state in which the gingival portion of the implantation target portion is hidden by the inner surface portion side of the wax bite for scanning and the end of the matching target portion is partially hidden by the outer surface portion side is shown as an image. Here, the third scanned image m13 is obtained while the implantation target portion and the matching target portion are occluded with each other through the wax bite for scanning. Accordingly, in the third scanned image m13, three-dimensional surface information corresponding to the wax bite for scanning and three-dimensional surface information t13 corresponding to an outer surface of a labial side and a partial outer surface of a buccal side of the matching tooth may be shown as an image.

Here, the plurality of scanned images may be obtained by directly scanning the oral cavity of the person to be treated, using the intraoral scanner, before and after installing the wax bite for scanning. In this case, a traction device configured to pull soft tissue, such as the lips and buccal mucosa, to be spaced apart from an outside of the gingival portion may be used. However, in some cases, the plurality of scanned images may be obtained by scanning impression models before and after installing the wax bite for scanning.

Meanwhile, the CT image m16 (refer to FIG. 7) may be obtained by directly capturing, using the CT apparatus, an image of the oral cavity while the wax bite for scanning is installed and occluded. Accordingly, in the CT image m16 (refer to FIG. 7), three-dimensional data and the like of the matching tooth of the matching target portion side and three-dimensional data of the alveolar bone of the implantation target portion side excluding soft tissue, such as the lips, buccal mucosa, gingivae, and the like, are imaged and displayed.

Furthermore, the wax bite for scanning may be formed in a preset color to be clearly displayed in the motion image and the plurality of scanned images. Also, the wax bite for scanning may be formed to have a density less than 2.0 g/cm³ to be passed through when the CT image m16 (refer to FIG. 7) is obtained and not to be displayed therein. Accordingly, the CT image m16 (refer to FIG. 7) obtained while the wax bite for scanning is installed and occluded is considered to have the occlusal vertical dimension while the alveolar bone of the implantation target portion side and the matching tooth/alveolar bone and the like of the matching target portion side are clearly displayed. Accordingly, accuracy and precision of each piece of image data obtained using the image capturing device may be significantly improved.

The plurality of scanned images and the CT image m16 (refer to FIG. 7) obtained as described above are transmitted to the planning portion. Also, through the following image alignment and matching process, the plurality of scanned images and the CT image m16 may be collected as information for establishing a tooth restoration plan using the dental restoration.

Meanwhile, the motion image is obtained by motion-scanning the three-dimensional motion track of the temporomandibular joint of the person to be treated using the dynamic scanner while the matching target portion and the masticatory protruding portion provided in the wax bite for scanning are occluded with each other.

In detail, referring to FIG. 6, the motion image c30 is obtained, using the dynamic scanner, with respect to the three-dimensional motion track of the temporomandibular joint while the outer surface of the oral cavity is exposed and the masticatory protruding portion 316 and the matching target portion 3 are occluded with each other. That is, a three-dimensional motion track of movement of a temporomandibular joint j (refer to FIG. 7) which moves in a left-right direction, a front-rear direction, and a vertical direction in the maximum movement radius g 1 is obtained through motion scanning using the dynamic scanner. Here, the dynamic scanner means a scanner provided to obtain a motion with respect to a three-dimensional motion track of a temporomandibular joint on the basis of a reference point such as an upper end point or the like of the interdentium corresponding portion 316b of the wax bite 310B for scanning when movement of the temporomandibular joint occurs. Here, the lips may be fixed while being opened using an additional traction device (not shown) in order to expose the outer surface of the oral cavity in which the wax bite 310B is installed.

Subsequently, while the outer surface of the oral cavity is exposed, the dynamic scanner may motion-scan movement of the temporomandibular joint in the left-right direction, front-rear direction, and vertical direction on the basis of the upper end point or the like of the interdentium corresponding portion 316b. Here, unlike scanning in which still image data is obtained, motion-scanning may be understood as a concept in which a track moved from a particular reference point is obtained on the basis of the reference point when movement of an object occurs.

Accordingly, unlike a conventional case in which an additional tracing plate is mounted on an oral cavity to obtain a gothic arch, the motion image c30 of the three-dimensional motion track of the temporomandibular joint which moves is digitalized and quickly obtained using the dynamic scanner. Accordingly, a working time may be significantly reduced and convenience in treatment may be significantly improved.

Also, the motion image c30 may be obtained using the dynamic scanner while the wax bite 310B is heated to a preset temperature or higher and softened. In detail, the motion image c30 may be obtained while the masticatory protruding portion 316 and the matching target portion 3 which have been heated to the preset temperature or higher and softened are occluded with each other. Accordingly, when the motion image c30 of the three-dimensional motion track of the temporomandibular joint is obtained while the wax bite for scanning is heated to the preset temperature or higher and softened, a space between the matching target portion 3 and the occlusal surface of the masticatory protruding portion 316 becomes lubricative to move flexibly. Accordingly, accuracy of the motion image c30 obtained using the dynamic scanner may be significantly improved, and the masticatory protruding portion 316 may be corrected as the occlusal correction portion 312r (refer to FIG. 10) corresponding to the three-dimensional motion track of the temporomandibular joint.

Accordingly, when the motion image c30 of the three-dimensional motion track of the temporomandibular joint is obtained while the wax bite 310B for scanning has been heated to the preset temperature or higher and softened, a caught portion between the matching target portion 3 and the occlusal surface of the masticatory protruding portion 316 moves. Accordingly, the accuracy of the motion image c30 obtained using the dynamic scanner may be significantly improved.

Meanwhile, the plurality of scanned images are aligned and arranged to correspond to the occlusal vertical dimension through the planning portion so that the integrated scanned image is generated. Also, the integrated scanned image is overlapped and matched with the CT image on the basis of a common part so that the three-dimensional planning image is generated (s320 in FIG. 1).

Here, referring to FIGS. 7 to 11B, integrated scanned image m17 may be generated through the following operations. In detail, the first scanned image m11 and the second scanned image m12 are overlapped and aligned on the basis of a common part with the third scanned image m13. Here, common parts of the respective scanned images may be set as particular parts having hard tissue with minimized mobility caused by an external force and having a boundary noticeably divided from a periphery thereof. For example, in the first scanned image m11 and the third scanned image m13, particular parts of three-dimensional surface information v11 and v13 of the wax bite for scanning displayed equally in the respective images may be selected as common parts and set to be overlapping reference points. Also, in the second scanned image m12 and the third scanned image m13, particular parts of the three-dimensional surface information t12 and t13 of the matching tooth displayed equally in the respective images may be selected as common parts and set to be overlapping reference points.

A plurality of such overlapping reference points may be set so that a shape-matching rate during a process of overlapping and arranging images may be significantly improved. Accordingly, the first scanned image m11 and the second scanned image m12 are aligned and arranged in consideration of the occlusal vertical dimension on the basis of the third scanned image m13.

Meanwhile, the first scanned image m11 may be swapped to externally expose the three-dimensional surface information m311r of the shape-matching correction portion concavely recessed inward, which corresponds to the inner surface portion of the wax bite for scanning. Here, swapping may be preferably understood as substituting and exchanging a preset image with another image or a modified image according to image processing. Accordingly, the three-dimensional surface information m311r of the shape-matching correction portion and the three-dimensional surface information on the outer surface of the matching target portion are aligned and arranged in a state in which the occlusal vertical dimension is considered to be generated as the integrated scanned image m17.

In detail, the first scanned image m11 may include three-dimensional surface information on an overall outer surface of the wax bite for scanning in which the inner and outer surface portions are corrected by the occlusal pressure. Hereinafter, the three-dimensional surface information of the wax bite for scanning may be understood as three-dimensional surface information of the wax bite for scanning in the state in which the inner and outer surface portions are corrected.

The three-dimensional surface information of the wax bite for scanning is stored while a plurality of points having preset coordinate values are mutually connected. For example, the three-dimensional surface information of the wax bite for scanning may be stored as a StereoLithography (STL) file and form a triangular surface by the plurality of points and lines connecting the same corresponding to an outer surface shape of the wax bite for scanning. Accordingly, the three-dimensional surface information of the wax bite for scanning may be stored as a three-dimensional surface model having a substantially hollow interior.

Here, the three-dimensional surface information of the wax bite for scanning includes the three-dimensional surface information m312r of the occlusal correction portion on the outer surface portion side displayed to be convex outward. Also, the three-dimensional surface information of the wax bite for scanning includes the three-dimensional surface information m311r of the shape-matching correction portion on the inner surface portion side displayed to be concave inward.

Here, a boundary line x is set along an outer edge of the three-dimensional surface information m311r of the shape-matching correction portion, and three-dimensional surface information of the masticatory protruding portion side is set to be a deletion area d. Also, an image is swapped so that the deletion area d is deleted and the three-dimensional surface information m311r of the shape-matching correction portion is exposed to be convex toward the matching target portion side. Here, the outer edge of the three-dimensional surface information m311r of the shape-matching correction portion may be understood as a line set corresponding to an outermost edge of the margin 211a. Here, in the present invention, "being deleted" may be understood as including deleting and treating an image selected from overall images and data to be transparent and invisible.

Meanwhile, the three-dimensional surface information of the wax bite for scanning is stored as surface information without a substantial thickness. Accordingly, the three-dimensional surface information m311r of the shape-matching correction portion has coordinate values for an inner surface side profile and coordinate values for an outer surface side profile which are substantially equal. Also, the three-dimensional surface information m311r of the shape-matching correction portion is obtained while being corrected to substantially shape-match and correspond to an outer surface profile of the implantation target portion. Accordingly, the three-dimensional surface information m311r of the shape-matching correction portion substantially corresponds to the outer surface profile of the gingival portion of the implantation target portion. Here, part m21 shown in FIGS. 7 and 11B may be understood as meaning the first scanned image swapped while the three-dimensional surface information m311r of the shape-matching correction portion is exposed outward.

The first scanned image m11 including the three-dimensional surface information of the wax bite for scanning is swapped so that the three-dimensional surface information on the outer surface profile of the gingival portion of the implantation target portion may be easily obtained. In addition, instead of the oral cavity with high fluidity of the gingiva that is soft tissue, three-dimensional surface information corresponding to the implantation target portion is obtained from the scanned image of the wax bite for scanning having firm strength in a hardened state although having fluidity correctable by the occlusal pressure in a softened state. Accordingly, reliability of image information for designing the dental restoration may be significantly improved. Here, to clearly display the second scanned image m12 and the swapped first scanned image m21, the third scanned image m13 may be deleted from the integrated scanned image m17.

Meanwhile, referring to FIG. 7, a part commonly displayed in the integrated scanned image m17 and the CT image m16 is set to be a matching reference point so that the integrated scanned image m17 and the CT image m16 are overlapped and matched. Accordingly, there is generated a three-dimensional planning image m20 including the three-dimensional surface information m311r of the shape-matching correction portion displayed in the swapped first scanned image m21, the three-dimensional data a16 of the alveolar bone on the implantation target portion side, and the three-dimensional surface information t12 and three-dimensional data of the matching tooth. Here, the three-dimensional data of the matching tooth displayed in the CT image m16 may be matched with the three-dimensional surface information t12 of the matching tooth displayed in the integrated scanned image m17 to be integrally and substantially overlapped therewith.

Here, the plurality of scanned images are aligned on the basis of the third scanned image obtained by scanning while occluding using the wax bite for scanning, and the CT image m16 is obtained while occluding using the wax bite for scanning. Accordingly, the three-dimensional surface information and three-dimensional data which are displayed in the CT image m16 and the integrated scanned image m17 are aligned and matched with each other corresponding to substantially the same occlusal vertical dimension VD. Accordingly, initial planning data in which an error in matching the integrated scanned image m17 with the CT image m16 is minimized may be obtained. Accordingly, the respective pieces of image data may be easily matched and a matching process may be simplified while accuracy and precision of collected information may be significantly improved. A height of the dental restoration is set on the basis of the three-dimensional planning image m20 generated as described above. Also, design information c20 of the dental restoration having a masticating surface shape set in consideration of the three-dimensional surface information t12 of the matching tooth is virtually disposed in the three-dimensional planning image m20.

Also, an implantation position and a direction h20 of the fixture may be set through the three-dimensional data a16 of the alveolar bone on the implantation target portion side based on the CT image m16. Also, a virtual fixture f20 supporting a masticating pressure is virtually disposed corresponding to the implantation position and the direction h20 while a real fixture corresponding to the virtual fixture f20 may be prepared or manufactured. In addition, a real abutment mediating between the real fixture and a real dental restoration manufactured on the basis of the design information c20 of the dental restoration may be prepared or manufactured.

Meanwhile, the occlusal vertical dimension is considered on the basis of the three-dimensional planning image m20, and the design information c20 of the dental restoration which includes a virtual masticating surface is generated. Also, the motion image is overlapped with the design information c20 of the dental restoration so that an overlapped occluding region c31 is displayed on the virtual masticating surface (s330 in FIG. 1).

In detail, as shown in FIGS. 7 and 8, the overlapped occluding region c31 may be set and displayed on the virtual masticating surface of the virtual artificial crown designed to be included in the design information c20 of the dental restoration and occluded with the matching target portion. Also, the design information c20 of the dental restoration from which the virtual masticating surface is extracted in consideration of the three-dimensional surface information t12 of the matching tooth is virtually disposed in the three-dimensional planning image m20. Also, the motion image is overlapped with the design information c20 of the dental restoration so that the overlapped occluding region c31 is displayed.

Also, the design information c20 of the dental restoration virtually disposed in the three-dimensional planning image m20 may be virtually moved along a three-dimensional motion track including a left-right direction, front-rear direction, and vertical direction on the basis of the motion image through the planning portion. Here, a region overlapped with the three-dimensional surface information t12 on the matching tooth of the matching target portion when the design information c20 of the dental restoration is virtually moved by the planning portion may be set and displayed as the overlapped occluding region c31.

Here, the overlapped occluding region c31 may be understood as data with respect to a three-dimensional region in which the design information c20 of the dental restoration is overlapped with the matching target portion when the virtual masticating surface moves along the three-dimensional motion track. Also, the overlapped occluding region c31 may be displayed with a different color from colors of a residual tooth and oral tissue in the planning portion.

Also, an overlapped deletion region c31a of the overlapped occluding region c31 is set such that the design information c20 of the dental restoration is corrected (s340 in FIG. 1). Here, setting of the overlapped deletion region c31a may be understood as deleting and treating images or data of the overlapped occluding region c31 to be transparent and invisible. Also, the overlapped deletion region c31a means a region set to have a gothic arch shape corresponding to the three-dimensional motion track on the virtual masticating surface.

Sequentially, corrected design information c20a of the dental restoration is transmitted from the planning portion to the manufacturing device and a real dental restoration is manufactured (s340 in FIG. 1). Here, the three-dimensional planning image m20 including the corrected design information c20a of the dental restoration may be stored as a standard file of the three-dimensional printer such as the above-described STL file. Accordingly, the real dental restoration precisely corresponding to the corrected design information c20a of the dental restoration may be easily manufactured. However, the real dental restoration may be manufactured using a milling device or a molding device.

Accordingly, the design information c20 of the dental restoration which is virtually disposed in the three-dimensional planning image m20 is moved along the three-dimensional motion track by the planning portion. Subsequently, since the three-dimensional region of the virtual masticating surface overlapped with the matching target portion is deleted, the design information c20 of the dental restoration is precisely corrected. Accordingly, precision of manufacturing a masticating surface of the finally manufactured dental restoration may be significantly improved. In addition, a processing time of the dental restoration actually manufactured on the basis of the design information c20a of the dental restoration which is corrected by deleting the overlapped deletion region c31a using the motion image c30 may be significantly reduced and working convenience may be significantly improved.

Also, a virtual masticating surface of the design information c20a of the dental restoration which is corrected by deleting the overlapped deletion region c31a may be set to be recessed like a gothic arch shape corresponding to the three-dimensional motion track of the temporomandibular joint.

Also, the masticating surface of the dental restoration manufactured on the basis of the corrected design information c20a of the dental restoration may be integrally manufactured to be recessed like the gothic arch shape. Here, the dental restoration means a real dental restoration corresponding to the design information c20a of the dental restoration, and the masticating surface of the dental restoration means a real masticating surface corresponding to the virtual masticating surface corrected by deleting the overlapped deletion region c31a.

Accordingly, the masticating surface of the dental restoration is manufactured on the basis of the design information c20a of the dental restoration which is corrected by setting and deleting the virtual masticating surface of the design information c20 of the dental restoration to be recessed like the gothic arch shape corresponding to the three-dimensional motion track of the temporomandibular joint. Accordingly, occlusal sensitivity of the finally-manufactured dental restoration may be significantly improved.

Also, the real masticating surface is manufactured on the basis of the design information c20a of the dental restoration which is corrected by setting and deleting the virtual masticating surface to be recessed like the gothic arch shape corresponding to the three-dimensional motion track of the temporomandibular joint. Accordingly, an additional operation of correcting the dental restoration which is necessary after final manufacturing may be minimized so as to significantly improve convenience of treatment.

As described above, in the present invention, an occlusal vertical dimension may be calculated using a simple method of applying occlusal pressure while the inner and outer surface portions of the wax bite 310, 310A, or 310B for scanning prepared as standardized ready-made articles of various sizes are heated. Accordingly, unlike the conventional case of using a splint which is customized and increases cost/time or a tracer which is inconvenient to operate, time and costs consumed for calculating the occlusal vertical dimension may be reduced while convenience in use may be significantly improved. Here, the wax bite 310, 310A, or 310B for scanning may be formed of paraffin wax to be easily deformed corresponding to the gingival portion g that is soft tissue when occluded at a temperature higher than or equal to a preset temperature as well as being easily cured so as to precisely guide an occlusal vertical dimension. Also, since it is possible to correct the wax bite 310, 310A, or 310B for scanning and obtain each piece of image data at one time when a person to be treated visits a dental clinic once, an overall period and cost of tooth restoration may be significantly reduced.

In addition, since occlusal pressure is uniformly transferred corresponding to an occlusal direction as the wax bite 310, 310A, or 310B for scanning is manufactured as one body using paraffin wax, distortion or damage may be minimized. Accordingly, the shape-matching correction portion 311r and the occlusal correction portion 312r may be accurately and precisely obtained on the inner and outer surface portions of the wax bite 310, 310A, or 310B for scanning so as to significantly improve reliability of information collected on the basis thereof. In addition, the shape-matching correction portion 311r is relined by curable resin and corrected to have a high shape-matching rate with the implantation target portion. Accordingly, instead of the implantation target portion 2, most of which is highly fluid gingival tissue, accuracy information may be obtained through scanned images of the shape-matching correction portion 311r. Accordingly, precision in designing and manufacturing the dental restoration may be significantly improved.

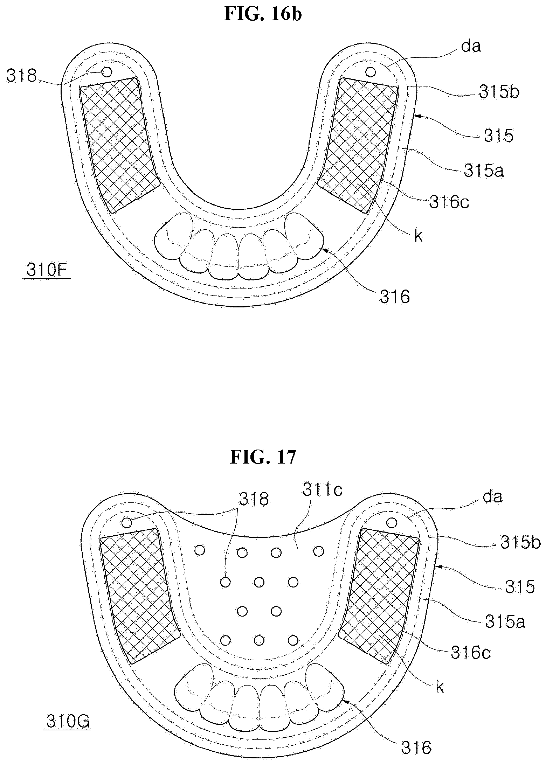

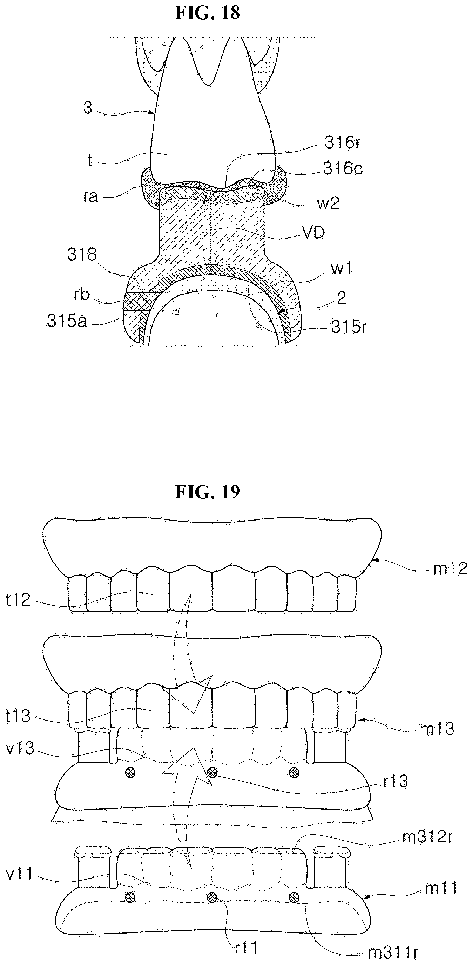

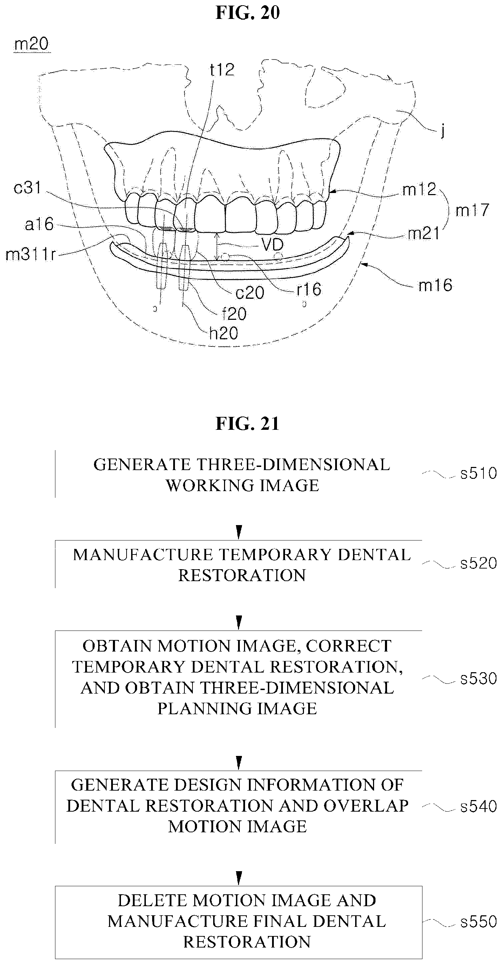

Meanwhile, FIGS. 16A and 16B are front and plan views in which a part of a wax bite for scanning according to a second embodiment of the present invention is projected, and FIG. 17 is a plan view illustrating a modified example of the wax bite for scanning according to the second embodiment of the present invention. Also, FIG. 18 is an exemplary cross-sectional view illustrating a process of correcting the wax bite for scanning according to the second embodiment of the present invention. Also, FIG. 19 is an exemplary view illustrating a process of obtaining an integrated scanned image using the wax bite for scanning according to the second embodiment of the present invention. Also, FIG. 20 is an exemplary view illustrating a process of overlapping and matching a CT image obtained using the wax bite for scanning according to the second embodiment of the present invention with the integrated scanned image. Here, since basic components of the second embodiment of the present invention are equal to those of the above first embodiment, a detailed description on the same components will be omitted and like components will be referred to as the same reference numerals.

As shown in FIGS. 16A to 20, wax bite 310G for scanning according to a modified example of the second embodiment of the present invention may further include a reinforcing surface portion 311c integrally connected to interiors of the variable shape-matching portion 315 and the masticatory protruding portion 316 and formed to support a space between both ends of the variable shape-matching portion 315 and the masticatory protruding portion 316.

In detail, the reinforcing surface portion 311c may be formed to support the space between both ends of masticatory protruding portion 316 and the variable shape-matching portion 315 formed to have an arch shape. Accordingly, even when the occlusal pressure is applied while the inner and outer surface portions of the wax bite 310G for scanning are softened so that supporting strength is reduced, distortion or fracture may be prevented.

Here, the wax bite 310G for scanning may be set to be curved in a standardized shape and accommodate a tongue so as to be manufactured for general use when applied to the lower jaw and may be manufactured for general use corresponding to a standard shape of the hard palate when applied to the upper jaw. Also, in a case of corresponding to the upper jaw side, since occlusion is corrected while the reinforcing surface portion 311c is supported by the hard palate, the wax bite 310G for scanning may be prevented from sliding and shifting in position in occlusion. Accordingly, the corrected wax bite 310G for scanning may be precisely corrected to accurately guide the occlusal vertical dimension.

Meanwhile, referring to FIGS. 16A to 17, a plurality of filling holes 318 may be formed in the recognition degradation surface of the wax bite for scanning to allow the curable resin to fill the plurality of filling holes 318 and be cured. Here, the recognition degradation surface means a smooth portion of a surface including a front surface portion of the wax bite 310F or 310G for scanning. Here, a rate of obtaining image data of the wax bite for scanning in oral scanning or motion scanning may be degraded due to the recognition degradation surface.

In detail, the plurality of filling holes 318 may be formed to pass through the variable shape-matching portion 315 and the reinforcing surface portion 311c to provide a matching reference point upon matching image data pieces obtained through oral scanning. Accordingly, the filling holes 318 formed in the recognition degradation surface of the wax bite 310F or 310G for scanning are filled with the curable resin so that image data of the matching reference point is provided when the scanned images and CT images obtained. Simultaneously, a motion recognition rate is reinforced when a gothic arch motion image of movement of the temporomandibular joint is obtained. Accordingly, since a following correction operation for a masticating surface of an artificial crown designed and manufactured on the basis of the motion image is minimized, convenience in treatment may be significantly improved.

Here, the filling holes 318 may be formed at a plurality of places at preset intervals in a front-rear direction and a vertical direction to pass through the variable shape-matching portion 315 and the reinforcing surface portion 311c. Also, any one of the filling holes 318 formed to pass through the front surface portion side of the variable shape-matching portion 315 may be formed in a central portion of a front surface of the oral cavity corresponding to the interdentium corresponding portion 316b, the maxillary labial frenum, or the mandibular labial frenum. Also, the plurality of filling holes 318 may be arranged at preset intervals in each of both directions from the filling hole 318 formed in the central portion of the front surface of the oral cavity.

Also, in some cases, intervals between the filling holes 318 formed in the front surface portion side of the variable shape-matching portion 315 in the recognition degradation surface may be arranged at an interval less than a maximum movement radius of the temporomandibular joint. Here, when the interval between the filling holes 318 exceeds the maximum movement radius of the temporomandibular joint, a motion track may not be recognized due to being the same color and the smooth surface structure of the wax bite 310F for scanning in motion-scanning of the movement of the temporomandibular joint using the dynamic scanner. That is, when the dynamic scanner scans the movement of the temporomandibular joint, a dynamic image obtained by scanning a motion change of a recognition degradation surface of the wax bite for scanning is converted into a three-dimensional modeling file. Here, a recognition rate of the dynamic scanner is degraded due to the smooth recognition degradation surface during an image conversion process. Accordingly, the intervals between the filling holes 318 formed in the recognition degradation surface are arranged at an interval less than the maximum movement radius of the temporomandibular joint so that the motion recognition rate of the dynamic scanner may be reinforced and a precise motion image may be obtained.

Meanwhile, the curable resin may be filled and cured so as to provide the matching reference point on the occlusal surface of the masticatory protruding portion 316 and the filling holes 318 and allow them to be relined corresponding to the occlusal vertical dimension VD. That is, the curable resin may be used for providing the matching reference point and calculating the occlusal vertical dimension VD.

In detail, referring to FIG. 18, the curable resin rb may be provided to have a different color from a color of the wax bite 310F for scanning. For example, when the wax bite 310F for scanning is provided to have a color similar to a color of the gingival portion of the person to be treated, the curable resin may be provided to have bluish colors and the like.

Accordingly, when a scanned image is obtained using the oral scanner while the wax bite 310F for scanning is disposed between the implantation target portion 2 and the matching target portion 3, the matching reference point may be displayed in a different color. Here, the scanned image means three-dimensional surface information on the implantation target portion 2, the matching target portion 3, and an outer surface of the wax bite 310F for scanning which are obtained using the oral scanner. Accordingly, the matching reference point may be displayed by the curable resin rb injected into the filling hole 318 and a plurality of scanned images which will be described below are matched corresponding to the occlusal vertical dimension VD so as to generate the integrated scanned image having a high reliability.

Also, the curable resin rb may be cured while filling in each of the filling holes 318 formed in the recognition degradation surface. Here, the curable resin rb filling in the filling holes 318 may include a radiopaque material to be image-captured in obtaining the CT image and provide the matching reference point. Here, the curable resin rb including the radiopaque material may be separately injected, using an additional curable resin filling device, into each of the filling holes 318 formed to pass through the variable shape-matching portion 315 and the reinforcing surface portion 311c. Here, the curable resin rb including the radiopaque material may be provided as a product or the like such as Blu-Mousse and the like of Parkell, Inc. but is not limited thereto.

Also, a CT image is obtained while the wax bite 310F for scanning with the filling holes 318 being filled with the cured curable resin rb including the radiopaque material is disposed between the implantation target portion 2 and the matching target portion 3. Here, the CT image means internal tissue information on shapes, densities, and the like of alveolar bones of the upper and lower jaws which are obtained using a CT apparatus. Here, since the curable resin rb includes the radiopaque material, an overall area where the curable resin rb fills and cured is displayed in the CT image. Accordingly, the area where the curable resin rb including the radiopaque material fills and is cured may be image-captured and displayed so as to provide the matching reference point.

Accordingly, the matching reference point is exposed and displayed in the scanned image and the CT image due to the curable resin rb formed of the radiopaque material injected into the filling holes 318 formed at the plurality of places passing through the recognition degradation surface. That is, the CT image and the integrated scanned image in which a plurality of scanned images, which will be described below, are matched may be easily matched by aligning respective matching reference points. Accordingly, a reliability of a three-dimensional planning image generated by matching the CT image and the integrated scanned image may be significantly improved so as to significantly improve precision of a finally manufactured dental restoration.

Also, referring to FIG. 18, the curable resin ra may be filled and cured to be relined on the outer surface 316c of the masticatory protruding portion on the molar tooth portion side corresponding to the occlusal vertical dimension VD of the person to be treated. Here, the masticatory protruding portion 316 may be formed to protrude with a cross-sectional area corresponding to a preset standard dental arch profile while the curable resin ra may be formed as a correction protruding portion cured while filling in the occlusal surface of the masticatory protruding portion 316. Here, the correction protruding portion means a finally corrected state as the curable resin ra fills in the occlusal surface of the masticatory protruding portion 316 and is cured.

Accordingly, an interval between the matching target portion 3 and the masticatory protruding portion 316 on the occlusal surface of the masticatory protruding portion 316 is corrected using the correction protruding portion so that the occlusal vertical dimension VD may be accurately calculated. Here, the friction marker k may be formed between the occlusal surface of the masticatory protruding portion 316 and the correction protruding portion so as to improve adhesion therebetween. Here, the curable resin ra filling in the outer surface 316c of the masticatory protruding portion may be selectively provided as any one of a radiation-transparent material or a radiopaque material. Accordingly, a three-dimensional planning image in which the precise occlusal vertical dimension VD is reflected is obtained through relining in which the curable resin ra fills and is cured between the masticatory protruding portion 316 and the matching target portion 3 corresponding to the occlusal vertical dimension of the person to be treated. Accordingly, precision of the finally-manufactured dental restoration may be significantly improved.

Meanwhile, referring to FIGS. 19 and 20, the first scanned image m11 may include a first matching reference image r 11 with respect to the matching reference point obtained by scanning the curable resin rb using the oral scanner. Also, the third scanned image m13 may include a second matching reference image r13 with respect to the matching reference point obtained by scanning the curable resin rb using the oral scanner. Also, the CT image m16 may include a third matching reference image r16 with respect to the matching reference point obtained using the curable resin rb (refer to FIG. 3) formed of the radiopaque material.

Also, the integrated scanned image m17 may be generated including operations as follows. In detail, the first scanned image m11 and the second scanned image m12 are overlapped and aligned on the basis of common parts with the third scanned image m13. Here, the first matching reference image r 11 of the first scanned image m11 and the second matching reference image r13 of the third scanned image m13 may be overlapped and aligned with each other. For example, the first matching reference image r11 and the second matching reference image r13 may be overlapped and aligned with each other. Meanwhile, a part commonly displayed in the integrated scanned image m17 and the CT image m16 is set to be a matching reference point so that the integrated scanned image m17 and the CT image m16 are overlapped and matched. Here, the third matching reference image r16 may be overlapped and aligned with an area in which the first matching reference image r11 and the second matching reference image r13 are overlapped.

Accordingly, there is generated a three-dimensional planning image m20 including the three-dimensional surface information m311r of the shape-matching correction portion displayed in the swapped first scanned image m21, the three-dimensional data a16 of the alveolar bone on the implantation target portion side, and the three-dimensional surface information t12 and three-dimensional data of the matching tooth. Here, the three-dimensional data of the matching tooth displayed in the CT image m16 may be matched with the three-dimensional surface information t12 of the matching tooth displayed in the integrated scanned image m17 to be integrally and substantially overlapped therewith.

Here, the plurality of scanned images are aligned on the basis of the third scanned image obtained by scanning while occluding using the wax bite for scanning, and the CT image m16 is obtained while occluding using the wax bite for scanning. Accordingly, the three-dimensional surface information and three-dimensional data which are displayed in the CT image m16 and the integrated scanned image m 17 are aligned and matched with each other corresponding to substantially the same vertical dimension VD. Accordingly, initial planning data in which an error in matching the integrated scanned image m17 with the CT image m16 is minimized may be obtained. Accordingly, the respective pieces of image data may be easily matched and a matching process may be simplified while accuracy and precision of collected information may be significantly improved.

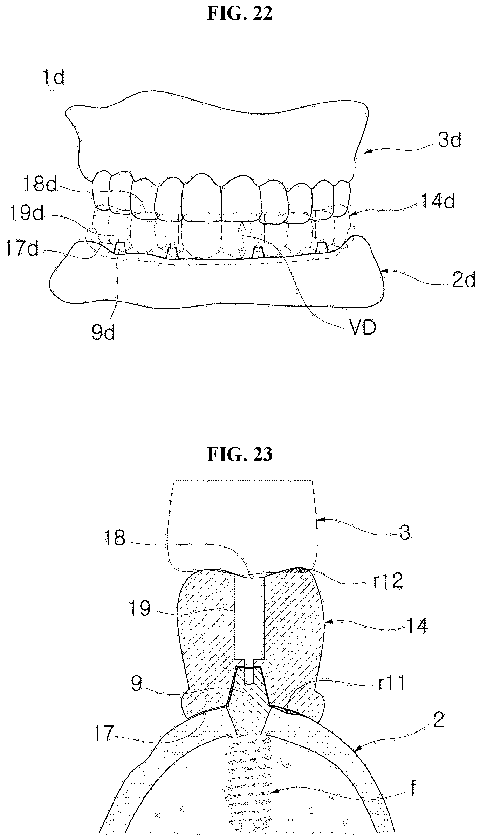

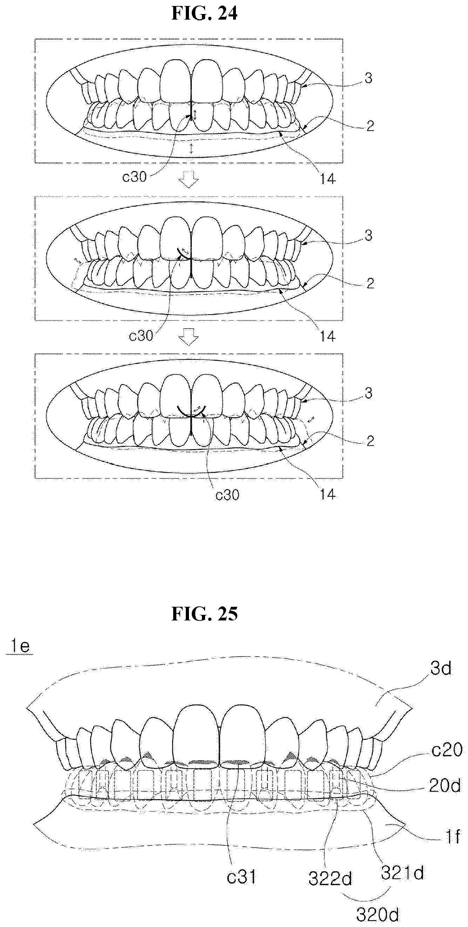

Meanwhile, FIG. 21 is a flowchart illustrating a dental restoration manufacturing method according to the second embodiment of the present invention, and FIG. 22 is an exemplary view illustrating a process of designing a temporary dental restoration in the dental restoration manufacturing method according to the second embodiment of the present invention. Also, FIG. 23 is an exemplary view illustrating a process of correcting the temporary dental restoration in the dental restoration manufacturing method according to the second embodiment of the present invention, and FIG. 24 is an exemplary view illustrating a process of obtaining a motion image in the dental restoration manufacturing method according to the second embodiment of the present invention. Also, FIG. 25 is an exemplary view illustrating a process of designing a metal frame and an artificial tooth in the dental restoration manufacturing method according to the second embodiment of the present invention. Here, in the second embodiment of the present invention, since basic components excluding a component configured to motion-scan a manufactured temporary dental restoration are equal to those of the above first embodiment, a detailed description on the same components will be omitted.

As shown in FIGS. 21 to 25, the dental restoration manufacturing method according to the second embodiment of the present invention includes a series of operations of generating a three-dimensional working image (s510), manufacturing a temporary dental restoration (s520), obtaining a motion image, correcting the temporary dental restoration, and obtaining a three-dimensional planning image (s530), generating design information of a dental restoration and overlapping the motion image (s540), and deleting the motion image and manufacturing a final dental restoration (s550).

In detail, a scanned image and a CT image of the implantation target portion and the matching target portion are aligned and matched on the basis of image information on the coupling bite configured to guide the occlusal vertical dimension for each person to be treated so as to generate a three-dimensional working image 1d (s510). Here, in the three-dimensional working image 1d, target surface information 2d corresponding to an outer surface profile of the implantation target portion side and matching surface information 3d corresponding to an outer surface profile of the matching target portion side are three-dimensionally imaged and displayed. In addition, there is included information on placing an implant such as a fixture, an abutment, or the like which is implanted to fix the dental restoration to an alveolar bone.

Also, in the three-dimensional working image 1d, the target surface information 2d in which a profile 9d of the abutment coupling portion is displayed and the matching surface information 3d spaced apart therefrom corresponding to the occlusal vertical dimension VD are imaged and displayed. In addition, when the implant is not placed in the implantation target portion side, three-dimensional data on the alveolar bone of the implantation target portion side is matched with the target surface information and displayed.

Also, a virtual temporary dental restoration 14d that includes design information of the temporary dental restoration is generated in the three-dimensional working image 1d including the target surface information 2d, the matching surface information 3d, and the implantation information (s520). Here, the temporary dental restoration is a dental restoration primarily manufactured using paraffin wax. to obtain precise design information on the finally manufactured dental restoration, and the dental restoration may be understood as a final dental restoration to be substantially used by a person to be treated.

In detail, the virtual temporary dental restoration 14d is generated in the three-dimensional working image 1d while a virtual occlusal surface portion 18d correspondingly occluded with a matching tooth remaining in the matching target portion side may be set on an outer surface portion of the virtual temporary dental restoration 14d. The virtual occlusal surface portion 18d may be set on the basis of the alignment of the matching tooth and three-dimensional masticating surface information to be stably occluded with the matching tooth. Also, a virtual shape-matching groove 17d may be set on an inner surface portion of the virtual temporary dental restoration 14d to be shape-matched with the target surface information 2d. The virtual shape-matching groove 17d may be set on the basis of the target surface information 2d to be substantially shape-matched with the implantation target portion. Here, in the target surface information 2d, the profile 9d of the abutment coupling portion corresponding to the implantation information is displayed while protruding.

Also, referring to FIGS. 22 and 23, a real temporary dental restoration 14 is manufactured on the basis of the virtual temporary dental restoration 14d including the virtual occlusal surface portion 18d and the virtual shape-matching groove 17d (s520). Here, in the first embodiment of the present invention, the temporary dental restoration 14 may be integrally formed including a temporary gingival portion including a shape-matching groove 17 including a coupling area 19 and a temporary tooth portion including an occlusal surface portion 18. Here, the above-described temporary dental restoration 14, shape-matching groove 17, occlusal surface portion 18, and coupling area 19 may be understood as real articles manufactured corresponding to the virtual temporary dental restoration 14d, the virtual shape-matching groove 17d, the virtual occlusal surface portion 18d, and a virtual coupling area 19d, respectively.

Meanwhile, referring to FIG. 24, the temporary dental restoration 14 is installed between the implantation target portion 2 and the matching target portion 3. Also, a gothic arch motion image c30 of a three-dimensional motion track of the temporomandibular joint of the person to be treated is obtained while the occlusal surface portion 18 of the temporary dental restoration 14 is occluded with the matching target portion 3 (s530). Here, since a process of obtaining the motion image c30 is equal to that of the above first embodiment, a detailed description thereof will be omitted.

Also, referring to FIGS. 23 and 24, the temporary dental restoration 14 may be installed between the implantation target portion 2 in which the fixture f and the abutment 9 are implanted and the matching target portion 3 occluded therewith. Here, the shape-matching groove 17, which is an inner surface portion side of the temporary dental restoration 14, is corrected to be shape-matched with the implantation target portion 2 in which the abutment 9 is implanted by occlusal pressure. In addition, the occlusal surface portion 18, which is an outer surface portion side of the temporary dental restoration 14, is corrected to correspond to an end of the matching tooth due to the occlusal pressure.

Also, the temporary dental restoration 14 corrected to match the shape-matching groove 17 and the occlusal surface portion 18 to be matched with the implantation target portion 2 and the matching target portion 3 due to the occlusal pressure may be scanned so as to obtain a three-dimensional planning image 1f of the temporary dental restoration 14. Subsequently, a shape-matching corrected surface portion formed by curing and fixing the curable resin may be included so as to correct the inner surface portion side of the temporary dental restoration 14, that is, the shape-matching groove 17.

Also, the occlusal surface portion 18 may be cut on the basis of an impression of the matching tooth of a side of the matching target portion 3 which is formed by the occlusal pressure and be adjusted including an occlusal corrected surface portion r12 to be optimized according to masticating sensitivity of the person to be treated. In addition, since the curable resin is further deposited outside the occlusal surface portion 18, an area of the occlusal corrected surface portion r12 may be increased. Accordingly, a height and a masticating surface of the artificial tooth set on the basis of the outer surface portion side of the temporary dental restoration 14, which includes the occlusal corrected surface portion r12, that is, the occlusal surface portion 18, may be more precisely and accurately formed.

Meanwhile, referring to FIG. 25, the three-dimensional planning image 1f may be obtained by scanning inner and outer surface portions of the corrected temporary dental restoration including the shape-matching corrected surface portion and the occlusal corrected surface portion. Also, the target surface information 2d and the matching surface information 3d which are included in the three-dimensional working image 1d may be replaced with the three-dimensional planning image 1f. Here, the reference numeral shown as 1e in FIG. 25 may be understood as meaning the three-dimensional planning image 1e which replaces the target surface information 2d to correct. Subsequently, the virtual metal frame 320d which includes design information of the metal frame and the design information c20 of the dental restoration which is design information of the artificial tooth may be generated on the basis of the three-dimensional planning image 1e.

Meanwhile, referring to FIG. 25, the virtual metal frame 320d may be set as follows. In detail, the design information c20 of the dental restoration suitable for the oral cavity of the person to be treated is extracted from the digital library and virtually disposed in the three-dimensional planning image 1e. Here, the design information c20 of the dental restoration is provided as a one set formed by arranging a plurality of virtual tooth models corresponding to a preset dental arch line while a plurality of such pieces of the design information c20 may be prestored in the digital library corresponding to a variety of dental arch lines by age and gender. Also, the design information c20 of one dental restoration selected and extracted according to a selected item corresponding to an oral environment of the person to be treated may be virtually disposed in the three-dimensional planning image 1e while a size, position, angle, and the like may be adjusted in consideration of an occlusal relationship with the matching tooth. Also, the height of the design information c20 of the dental restoration may be set corresponding to the matching surface information 3d or the outer surface portion corrected surface information.

Also, a virtual gingiva extension portion 321d may be virtually disposed to entirely surround a bottom end of the design information c20 of the dental restoration, that is, an end on a dental root side. Here, an inner surface of the virtual gingiva extension portion 321d is virtually disposed according to the inner surface portion corrected surface information to correspond thereto. Accordingly, an inner surface of the virtual metal frame 320d corresponding to the inner surface of the virtual gingiva extension portion 321d may be set to be precisely installed in the implantation target portion in which the abutment is implanted. Here, a virtual support protrusion portion 322d protruding from the virtual gingiva extension portion 321d separately corresponding to the design information c20 of the dental restoration may be set. Here, the virtual metal frame 320d may be set by integrating the virtual gingiva extension portion 321d with the virtual support protrusion portion 322d. In addition, a virtual support groove portion may be set, corresponding to an outer surface of the virtual support protrusion portion 322d, in the design information c20 of the dental restoration.

Also, the virtual fastening hole may be virtually disposed to pass through the virtual gingiva extension portion 321d and the virtual support protrusion portion 322d corresponding to the implantation information. Here, the virtual stepped portion and the virtual coupling end may be set to be located inside the virtual support protrusion portion 322d while a virtual communication hole configured to communicate with an upper side of the virtual fastening hole may be set in the design information c20 of the dental restoration.

Meanwhile, referring to FIG. 25, the motion image c30 is overlapped with a virtual masticating surface of the design information c20 of the dental restoration so that the overlapped occlusion region c31 is displayed (s540). Here, the design information c20 of the dental restoration virtually disposed in the three-dimensional planning image 1f may be virtually moved along a three-dimensional motion track on the basis of the motion image c30 using the planning portion. Also, an area in which the design information c20 of the dental restoration and three-dimensional surface information on the virtual occlusal surface portion of the matching target portion 3 are overlapped by the planning portion may be set and displayed as the overlapped occlusion region c31.

Also, an overlapped deletion region of the overlapped occlusion region c31 is set to correct the design information c20 of the dental restoration and an artificial tooth manufactured corresponding to corrected design information of the dental restoration is coupled to the metal frame so as to manufacture a final dental restoration (s550). Here, setting of the overlapped deletion region may be understood as deleting and treating images or data of the overlapped occluding region c31 to be transparent and invisible. Also, the overlapped deletion region means a region set to have a gothic arch shape corresponding to the three-dimensional motion track on the virtual masticating surface.

Sequentially, the corrected design information of the dental restoration is transmitted from the planning portion to the manufacturing device and the real dental restoration is manufactured. Here, the three-dimensional planning image 1f including the corrected design information of the dental restoration may be stored as a standard file of the three-dimensional printer such as the above-described STL file. Accordingly, a real dental restoration precisely corresponding to the corrected design information of the dental restoration may be easily manufactured.

Accordingly, the design information c20 of the dental restoration which is virtually disposed in the three-dimensional planning image 1f is moved along the three-dimensional motion track by the planning portion. Subsequently, since the three-dimensional region of the virtual masticating surface overlapped with the matching target portion is deleted, the design information c20 of the dental restoration is precisely corrected. Accordingly, precision of manufacturing a masticating surface of the finally manufactured dental restoration may be significantly improved. In addition, a processing time of the dental restoration actually manufactured on the basis of the design information of the dental restoration which is corrected by overlapping and deleting using the motion image c30 may be significantly reduced and working convenience may be significantly improved.

Also, a virtual masticating surface of the design information of the dental restoration which is corrected by deleting the overlapped deletion region may be set to be recessed like a gothic arch shape corresponding to the three-dimensional motion track of the temporomandibular joint. Also, the masticating surface of the artificial tooth manufactured on the basis of the corrected design information of the dental restoration may be integrally manufactured to be recessed like the gothic arch shape. Here, the dental restoration means a real dental restoration corresponding to the design information c20a of the dental restoration, and the masticating surface of the dental restoration means a real masticating surface corresponding to the virtual masticating surface corrected by deleting the overlapped deletion region.

Accordingly, the masticating surface of the artificial tooth is manufactured on the basis of the design information of the dental restoration which is corrected by setting and deleting the virtual masticating surface of the design information c20 of the dental restoration to be recessed like the gothic arch shape corresponding to the three-dimensional motion track of the temporomandibular joint. Accordingly, occlusal sensitivity of the finally-manufactured dental restoration may be significantly improved.

Meanwhile, the metal frame and the artificial tooth are manufactured corresponding to the virtual metal frame set through the above operation and the design information of the dental restoration. Here, the metal frame and the artificial tooth may be understood as a real article manufactured corresponding to the virtual metal frame and the design information of the dental restoration. Also, a final dental restoration may be manufactured by coupling the metal frame to the artificial tooth.

In detail, the artificial tooth may be manufactured by three-dimensional printing or three-dimensional milling of a synthetic resin or zirconium to have a shape, rigidity, and the like similar to a real tooth. Here, a gingiva extension portion, a support protrusion portion, a fastening hole, a stepped portion, and a coupling end may be formed in the metal frame. Here, the above gingiva extension portion and support protrusion portion may be understood as real articles manufactured corresponding to the above virtual gingiva extension portion 321d and the virtual support protrusion portion 322d. Also, the above fastening hole, stepped portion, and coupling end may be understood as real articles manufactured corresponding to the above virtual fastening hole, virtual stepped portion, and virtual coupling end.

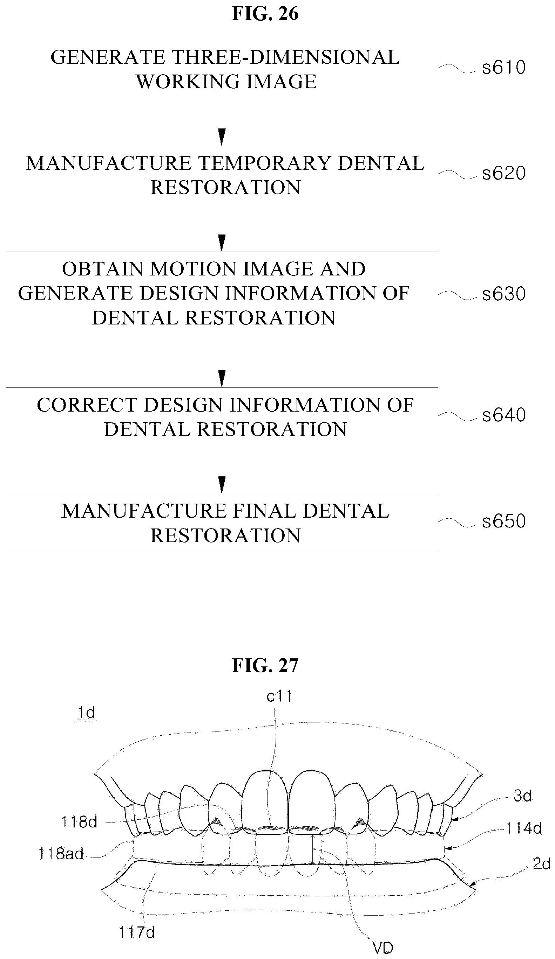

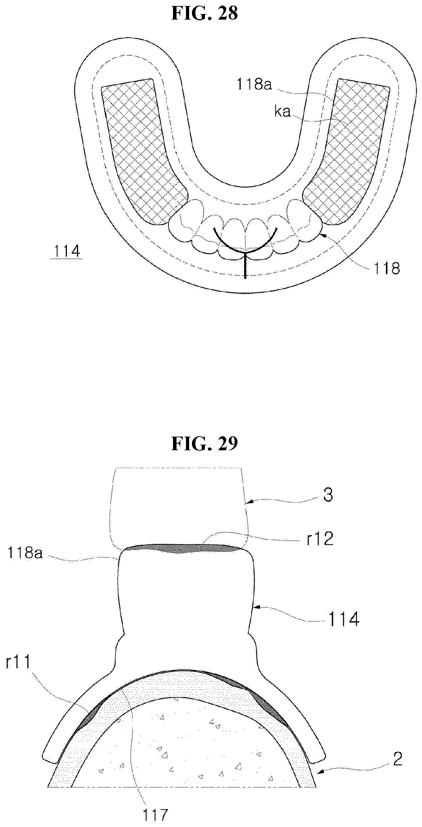

Meanwhile, FIG. 26 is a flowchart illustrating a dental restoration manufacturing method according to a third embodiment of the present invention, and FIG. 27 is an exemplary view illustrating a process of designing a temporary dental restoration in the dental restoration manufacturing method according to the third embodiment of the present invention. Also, FIG. 28 is an exemplary view illustrating a temporary dental restoration in a dental restoration according to the third embodiment of the present invention, and FIG. 29 is an exemplary view illustrating a process of correcting the temporary dental restoration in the dental restoration manufacturing method according to the third embodiment of the present invention. Here, since basic components of the third embodiment of the present invention are equal to those of the above second embodiment, a detailed description on the same components will be omitted and like components will be referred to as the same reference numerals.

As shown in FIGS. 26 to 29, the dental restoration manufacturing method according to the third embodiment of the present invention includes a series of operations of generating a three-dimensional working image (s610), manufacturing a temporary dental restoration (s620), obtaining a motion image and generating design information of a dental restoration (s630), correcting design information of the dental restoration (s640), and manufacturing a final dental restoration (s650).

Meanwhile, an operation of preliminarily obtaining a gothic arch preliminary motion image while the matching target portion 3 and a masticatory protruding portion provided in the wax bite 310 disposed between the implantation target portion 2 and the matching target portion 3 are occluded with each other may be performed. Here, the masticatory protruding portion means an area corresponding to the other surface portion.

Here, the preliminary motion image may be preliminarily obtained by motion-scanning a three-dimensional motion track with respect to movement of the temporomandibular joint in a left-right direction, front-rear direction, and vertical direction using the dynamic scanner. In detail, the preliminary motion image may be preliminarily obtained by motion-scanning the three-dimensional motion track of the temporomandibular joint of the person to be treated using the dynamic scanner while the masticatory protruding portion and the matching target portion 3 are occluded with each other. Here, a process of obtaining the preliminary motion image preliminarily obtained by motion-scanning the wax bite may be understood as substantially equal to a process of obtaining a motion image obtained by motion-scanning a temporary dental restoration excluding motion-scanning the wax bite.

Also, the preliminary motion image may be obtained using the dynamic scanner while the wax bite 310 is heated to a preset temperature or higher and softened. Accordingly, when the preliminary motion image of the three-dimensional motion track of the temporomandibular joint is obtained while the wax bite 310 is heated to the preset temperature or higher, a space between the matching target portion and the occlusal surface of the wax bite 310 becomes lubricated to move flexibly. Accordingly, accuracy of the preliminary motion image obtained using the dynamic scanner may be significantly improved.

Also, a virtual temporary dental restoration 114d that includes design information of the temporary dental restoration is generated in the three-dimensional working image 1d including the target surface information 2d and the matching surface information 3d. Here, the virtual temporary dental restoration and the design information of the temporary dental restoration may be understood to have the same meaning. Hereinafter, a case in which the temporary dental restoration manufactured corresponding to the virtual temporary dental restoration and the dental restoration manufactured corresponding to the design information of the dental restoration are manufactured as full dentures will be described and illustrated.

In detail, the virtual temporary dental restoration 114d is generated in the three-dimensional working image 1d while a virtual occlusal surface portion 118d correspondingly occluded with a matching tooth remaining in the matching target portion side may be set on an outer surface portion on an anterior tooth portion side of the virtual temporary dental restoration 114d. Here, the virtual occlusal surface portion 118d may be set on the basis of the alignment of the matching tooth and three-dimensional masticating surface information to be stably occluded with the matching tooth.

Here, the preliminary motion image may be overlapped with the virtual temporary dental restoration 114d using the planning portion so as to display a preliminary overlapped occlusion region c11 in the virtual occlusal surface portion 118d. Here, the preliminary overlapped occlusion region c11 means an area in which the preliminary motion image is overlapped with the virtual occlusal surface portion 118d. Also, an operation of preliminarily correcting the virtual occlusal surface portion 118d of the virtual temporary dental restoration 114d by setting a preliminary overlapped deletion region in the preliminary overlapped occlusion region c11 may be further performed.

Accordingly, the temporary dental restoration 114 precisely manufactured while the virtual occlusal surface portion 118d of the virtual temporary dental restoration 114d is preliminarily corrected on the basis of the preliminary motion image preliminarily obtained by motion-scanning the wax bite 310 is motion-scanned once more. Accordingly, precision of manufacturing a masticating surface of the finally manufactured dental restoration may be significantly improved by obtaining the motion image through multiple stages.

Also, a virtual temporary corrected surface portion 118ad correspondingly occluded with a matching tooth remaining on the matching target portion side and protruding with a cross-sectional area corresponding to a profile of a preset standard dental arch may be set on an outer surface portion on a molar tooth side of the virtual temporary dental restoration 114d. Here, a virtual temporary friction marker including at least one of a scratch and an intaglio may be set in an occlusal surface of the virtual temporary corrected surface portion 118ad. Also, a virtual shape-matching groove 117d may be set on an inner surface portion of the virtual temporary dental restoration 114d to be shape-matched with the target surface information 2d.

Also, a rear temporary dental restoration 114, in which the temporary corrected surface portion 118a is formed, is manufactured on the basis of the virtual temporary dental restoration 114d. Here, the temporary dental restoration 114 is manufactured on the basis of the virtual temporary dental restoration 114d including the virtual occlusal surface portion 118d, the virtual temporary corrected surface portion 118ad, and the virtual shape-matching groove 117d.

Here, the temporary dental restoration 114 may include a temporary tooth portion including an inner surface portion and an outer surface portion which are integrally formed. In detail, a shape-matching groove 117 manufactured corresponding to the target surface information 2d and shape-matched with the implantation target portion 2 may be formed in the inner surface portion. Also, an occlusal surface portion 118 manufactured corresponding to the matching surface information 3d and occluded with the matching target portion 3 may be formed on the outer surface portion. Here, the occlusal surface portion 118 may be understood as being manufactured on the basis of the virtual occlusal surface portion 118d preliminarily corrected by setting the preliminary overlapped deletion region with respect to the preliminary overlapped occlusion region c11.

In addition, the temporary corrected surface portion 118a in which a temporary friction marker ka is formed may be formed on the outer surface portion to protrude with a cross-sectional area corresponding to a preset standard dental arch profile and have improved adhesion. Here, the above-described temporary dental restoration 114, shape-matching groove 117, occlusal surface portion 118, temporary corrected surface portion 118a, and temporary friction marker ka may be understood as real articles manufactured corresponding to the virtual temporary dental restoration 114d, the virtual shape-matching groove 117d, the virtual occlusal surface portion 118d, the virtual temporary corrected surface portion 118ad, and the virtual temporary friction marker, respectively. Here, a curable resin filling and cured to be relined corresponding to an occlusal vertical dimension for each person to be treated may fills and is cured in the shape-matching groove 117, the occlusal surface portion 118, and the temporary corrected surface portion 118a.

Also, the temporary corrected surface portion 118a may be formed to protrude with a cross-sectional area corresponding to the preset standard dental arch profile. Also, the temporary friction marker ka including at least any one of a scratch and intaglio may be formed on the occlusal surface of the temporary corrected surface portion 118a to improve adhesion with the curable resin that fills in the occlusal surface while relining. Here, the curable resin may be cured while filling in the occlusal surface of the temporary corrected surface portion 118a so that a gap between the matching target portion 3 and the temporary corrected surface portion 118a may be formed as a temporary corrected protruding portion corrected corresponding to the occlusal vertical dimension VD.

Accordingly, the temporary friction marker ka may be formed, such as a scratch, intaglio, and the like, in the occlusal surface of the temporary corrected surface portion 118a. Accordingly, a surface area of the temporary corrected surface portion 118a increases when the curable resin fills in the occlusal surface of the temporary corrected surface portion 118a corresponding to the occlusal vertical dimension of the person to be treated. Accordingly, since adhesion between the occlusal surface of the temporary corrected surface portion 118a and the curable resin is improved, the three-dimensional planning image 1f on which a precise occlusal vertical dimension is reflected may be obtained.

Also, the occlusal surface portion 118 and the temporary corrected surface portion 118a may be cut on the basis of an impression of the matching tooth of a side of the matching target portion 3 which is formed by the occlusal pressure and be adjusted including an occlusal correction surface portion r12 to be optimized according to masticating sensitivity of the person to be treated. In addition, since the curable resin is relined and further deposited outside the occlusal surface portion 118 and the temporary corrected surface portion 118a, an area of the occlusal correction surface portion r12 may be increased. Accordingly, a height and a masticating surface of the artificial tooth set on the basis of the outer surface portion side of the temporary dental restoration 114, which includes the occlusal correction surface portion r12, that is, the occlusal surface portion 118 and the temporary corrected surface portion 118a, may be more precisely and accurately formed.

Here, since the term "comprise," "include," "have," or the like, unless particularly defined otherwise, means that a corresponding component can be included, it should be construed that another component is not excluded and may be further included. All the terms used herein, including technical or scientific terms, unless defined otherwise, have the same meanings generally understood by one of ordinary skill in the art. Generally used terms such as the terms defined in dictionaries should be understood as having meanings which coincide with contextual meanings of the related art and will not be understood as ideally or excessively formal meanings unless clearly defined.

The present invention may be applied to a dental restoration industry by providing a dental restoration manufacturing method with improved precision and reliability of a dental restoration.

## Claims

1. A dental restoration manufacturing method comprising:
a first operation of obtaining, using an image-capturing device (320), each of a scanned image of an implantation target portion (2) and a matching target portion (3) before installing a coupling bite (309) disposed between the implantation target portion (2) and the matching target portion (3) and after installing inner and outer surface portions of the coupling bite (309) through occlusal pressure in consideration of an occlusal vertical dimension of a person to be treated, a computerized tomography (CT) image (m16) of an oral cavity occluded using the coupling bite (309), and a gothic arch motion image (c30) of a three-dimensional motion track of a temporomandibular joint of the person to be treated while a masticatory protruding portion provided in the coupling bite and the matching target portion are occluded with each other, and transmitting the images to a planning portion (330);
a second operation of generating an integrated scanned image (m17) by aligning and arranging a plurality of such scanned images to correspond to the occlusal vertical dimension while generating a three-dimensional planning image (m20) by overlapping and matching the scanned images on the basis of common parts with the CT image (m16);
a third operation of generating and disposing design information (c20) of a dental restoration including a virtual masticatory surface on the basis of the three-dimensional planning image (m20) and overlapping the motion image (c30) with the design information (c20) of the dental restoration so as to display an overlapped occlusion region (c31) on the virtual masticatory surface; and
a fourth operation of correcting the design information (c20) of the dental restoration by setting an overlapped deletion region (c31a) with respect to the overlapped occlusion region (c31) and transmitting the corrected design information (c20) of the dental restoration to a manufacturing device (340) so as to manufacture the dental restoration.

2. The dental restoration manufacturing method of claim 1, wherein in the first operation, the motion image (c30) is obtained by motion-scanning, using a dynamic scanner, a three-dimensional motion track of movement of the temporomandibular joint in a left-right direction, front-rear direction, and vertical direction while an outer surface of the oral cavity is exposed and the masticatory protruding portion and the matching target portion are occluded with each other.

3. The dental restoration manufacturing method of claim 1, wherein the third operation comprises:
virtually moving the design information (c20) of the dental restoration which is virtually disposed on the three-dimensional planning image (m20) along the three-dimensional motion track on the basis of the motion image (c30) using the planning portion (330); and
setting and displaying, by the planning portion (330), an area in which the design information (c20) of the dental restoration and three-dimensional surface information (t12) on a matching tooth of the matching target portion are overlapped as the overlapped occlusion region (c31).

4. The dental restoration manufacturing method of claim 1, wherein in the first operation, the coupling bite is (309) provided as a wax bite (310) for scanning, and wherein the wax bite for scanning comprises:
a variable shape-matching portion (311, 313, 315) formed while being standardized corresponding to a preset standard dental arch profile and disposed between the implantation target portion (2) and the matching target portion (3) corresponding to the implantation target portion while being softened when heated to a preset temperature or higher; and
a masticatory protruding portion (316) integrally formed with the variable shape-matching (311, 313, 315) portion and protruding corresponding to the matching target portion (3) while being softened when heated to a preset temperature or higher.

5. The method of claim 4, wherein in the first operation, the wax bite (310) for scanning is pressurized so that a gap between the variable shape-matching portion (311, 313, 315) and the masticatory protruding portion (315), which are heated within a preset temperature range to be softened, corresponds to the occlusal vertical dimension while the variable shape-matching portion (311, 313, 315) having a margin greater than a width of a standard gingival portion is corrected to correspondingly shape-match with an outer surface profile of the implantation target portion (2) and integrally formed with a shape-matching correction portion (311r), and the masticatory protruding portion (315) is corrected to correspondingly occlude with the matching target portion (3) and integrally formed with an occlusal correction portion (312r) to be installed.

6. The dental restoration manufacturing method of claim 5, wherein in the first operation, the shape-matching correction portion (311r) is relined so that an inner surface is filled and occluded with a curable resin to compensate for a gap between the shape-matching correction portion (311r) and the implantation target portion (2).

7. The method of claim 5, wherein in the first operation, the scanned image comprises:
a first scanned image (m11) of the inner and outer surface portions of the wax bite (310) for scanning which is corrected to integrally form the shape-matching correction portion (311r) and the occlusal correction portion (312r);
a second scanned image (m12) of an outer surface of the matching target portion (3); and
a third scanned image (m13) of outer surfaces of the implantation target portion (2) and the matching target portion (3) which are occluded with the wax bite for scanning.

8. The dental restoration manufacturing method of claim 7, wherein in the first operation, a plurality of such scanned images further comprise a fourth scanned image of the outer surface of the implantation target portion (3),
wherein in the first operation, the integrated scanned image (m17) is generated by overlapping and aligning the first scanned image (m11) with the second scanned image (m12) on the basis of common parts with the third scanned image (m13) corresponding to the occlusal vertical dimension while swapping the first scanned image with the fourth scanned image, and
wherein in the second operation, the integrated scanned image (m17) is generated by overlapping and aligning the first scanned image (m11) with the second scanned image (m12) on the basis of each common part with the third scanned image (m13) corresponding to the occlusal vertical dimension while swapping the first scanned image (m11) to externally expose three-dimensional surface information of the shape-matching correction portion (311r) corresponding to an inner surface portion of the wax bite (310) for scanning.

9. A dental restoration manufacturing method comprising:
a first operation (s510) of generating a three-dimensional working image (1d) including target and matching surface information by aligning and matching a scanned image (m17) and a CT image (c16) of an implantation target portion (2) and a matching target portion (3) on the basis of image information on an occlusal vertical dimension for each person to be treated;
a second operation (s520) of generating a virtual temporary dental restoration (14d) in which inner and outer surface portions corresponding to the target and matching surface information are set in the three-dimensional working image (1d) and manufacturing a temporary dental restoration (14) on the basis of the virtual temporary dental restoration (14d), wherein said temporary dental restoration (14) is manufactured using paraffin wax;
a third operation (s530) of obtaining a gothic arch motion image (c30) of a three-dimensional motion track of a temporomandibular joint of the person to be treated while the temporary dental restoration is installed between the implantation target portion (2) and the matching target portion (3) and an occlusal surface portion (18) of the temporary dental restoration (14) is occluded with the matching target portion (3) and generating design information (c20) of a dental restoration including a virtual masticatory surface on the basis of a three-dimensional planning image (1f) obtained by correcting the target and matching surface information;
a fourth operation (s540) of correcting the design information (c20) of the dental restoration by overlapping the motion image (c30) with the design information (c20) of the dental restoration to display an overlapped occlusion region (c31) on the virtual masticatory surface and setting an overlapped deletion region (c31r) with respect to the overlapped occlusion region (c31); and
a fifth operation (s550) of manufacturing a final dental restoration by manufacturing an artificial tooth corresponding to the corrected design information (c20a) of the dental restoration.

10. The dental restoration manufacturing method of claim 9, wherein the fourth operation comprises setting the virtual masticatory surface of the corrected design information (c20a) of the dental restoration to be recessed to be a gothic arch shape corresponding to the three-dimensional motion track of the temporomandibular joint by deleting the overlapped deletion region (c31r), and
wherein the fifth operation comprises manufacturing a masticatory surface of the artificial tooth manufactured on the basis of the corrected design information (c20a) of the dental restoration to be recessed like the gothic arch shape.

11. The dental restoration manufacturing method of claim 9, wherein the fourth operation comprises:
virtually moving the design information of the dental restoration which is virtually disposed on the three-dimensional planning image (1f) along the three-dimensional motion track on the basis of the motion image using the planning portion (330); and
setting and displaying, by the planning portion (330), an area in which the design information of the dental restoration and three-dimensional surface information on a virtual occlusal surface portion of the matching target portion (3) are overlapped as the overlapped occlusion region (c31).

12. The dental restoration manufacturing method of claim 9, wherein in the third operation, the motion image (c30) is obtained by motion-scanning, using a dynamic scanner, a three-dimensional motion track of movement of the temporomandibular joint in a left-right direction, front-rear direction, and vertical direction while an outer surface of the oral cavity is exposed and the occlusal surface portion and the matching target portion are occluded with each other.

13. The dental restoration manufacturing method of claim 9, wherein in the second operation, a temporary corrected surface portion (118a) protruding with a cross-sectional area corresponding to a preset standard dental arch profile is formed on the temporary dental restoration (114) while a temporary friction marker including at least one of a scratch and an intaglio to improve adhesion with a curable resin that fills during relining is formed in an occlusal surface of the temporary corrected surface portion (118a), and
wherein in the third operation, the target and matching surface information is corrected by filling the temporary corrected surface portion (118a) with the curable resin and curing the curable resin while the curable resin is cured while filling in the occlusal surface of the temporary corrected surface portion (118a) so that a gap between the matching target portion (3) and the temporary corrected surface portion (118a) is formed as a temporary corrected protruding portion corrected corresponding to the occlusal vertical dimension.

14. The dental restoration manufacturing method of claim 9, wherein the first operation further comprises preliminarily obtaining a gothic arch preliminary motion image while a masticatory protruding portion provided in a wax bite (310) formed of a paraffin wax material disposed between the implantation target portion (2) and the matching target portion (3) and the matching target portion are occluded with each other, and
wherein the second operation further comprises preliminarily correcting the virtual dental restoration by displaying a preliminary overlapped occlusion region on a virtual occlusal surface portion (r12) of the virtual temporary dental restoration by overlapping the preliminary motion image with the virtual temporary dental restoration (114d) and setting a preliminary overlapped deletion region with respect to the preliminary overlapped occlusion region.

## Patentansprüche

1. Verfahren zur Herstellung eines Zahnersatzes, das umfasst:
einen ersten Vorgang zum Erhalten, unter Verwendung einer Bildaufnahmevorrichtung (320) von jeweils einem gescannten Bild eines Implantationszielabschnitts (2) und eines passenden Zielabschnitts (3) vor der Installation eines Kopplungsbisses (309), der zwischen dem Implantationszielabschnitt (2) und dem passenden Zielabschnitt (3) angeordnet ist, und nach der Installation innerer und äußerer Oberflächenabschnitte des Kopplungsbisses (309) durch okklusalen Druck unter Berücksichtigung einer okklusalen vertikalen Dimension einer zu behandelnden Person, eines computertomographischen (CT) Bildes (m16) einer Mundhöhle, die unter Verwendung des Kopplungsbisses (309) okkludiert ist, und eines Bewegungsbildes (c30) des gotischen Bogens einer dreidimensionalen Bewegungsspur eines Temporomandibulargelenks der zu behandelnden Person, während ein in dem Kopplungsbiss vorgesehener vorstehender Kauabschnitt und der passende Zielabschnitt miteinander okkludiert sind, und Übertragen der Bilder an einen Planungsabschnitt (330);
einen zweiten Vorgang zum Erzeugen eines integrierten gescannten Bildes (m17) durch Ausrichten und Anordnen einer Vielzahl solcher gescannter Bilder, so dass sie der okklusalen vertikalen Dimension entsprechen, bei gleichzeitigem Erzeugen eines dreidimensionalen Planungsbildes (m20) durch Überlappen und In-Übereinstimmung-Bringen der gescannten Bilder auf der Basis gemeinsamer Teile mit dem CT-Bild (m16),
einen dritten Vorgang zum Erzeugen und Bereitstellen von Konstruktionsinformationen (c20) eines Zahnersatzes einschließlich einer virtuellen Kaufläche auf der Grundlage des dreidimensionalen Planungsbildes (m20) und zum Überlappen des Bewegungsbildes (c30) mit den Konstruktionsinformationen (c20) des Zahnersatzes, um einen überlappenden Okklusionsbereich (c31) auf der virtuellen Kaufläche anzuzeigen; und
einen vierten Vorgang zum Korrigieren der Konstruktionsinformationen (c20) des Zahnersatzes durch Festlegen eines überlappenden Löschbereichs (c31a) in Bezug auf den überlappenden Okklusionsbereich (c31) und Übertragen der korrigierten Konstruktionsinformationen (c20) des Zahnersatzes an eine Herstellungsvorrichtung (340), um den Zahnersatz herzustellen.

2. Verfahren zur Herstellung von Zahnersatz nach Anspruch 1, wobei in dem ersten Vorgang das Bewegungsbild (c30) durch Bewegungsabtastung, unter Verwendung eines dynamischen Scanners, einer dreidimensionalen Bewegungsspur der Bewegung des Temporomandibulargelenks in einer Links-Rechts-Richtung, einer Vorne-Hinten-Richtung und einer vertikalen Richtung erhalten wird, während eine äußere Oberfläche der Mundhöhle freigelegt ist und der aus dem vorstehende Kauabschnitt und der passende Zielabschnitt miteinander okkludiert sind.

3. Verfahren zur Herstellung von Zahnersatz nach Anspruch 1, wobei der dritte Vorgang umfasst:
virtuelles Bewegen der Konstruktionsinformationen (c20) des Zahnersatzes, der virtuell auf dem dreidimensionalen Planungsbild (m20) angeordnet ist, entlang der dreidimensionalen Bewegungsspur auf der Basis des Bewegungsbildes (c30) unter Verwendung des Planungsabschnitts (330); und
Festlegen und Anzeigen, durch den Planungsabschnitt (330), eines Bereichs, in dem die Konstruktionsinformationen (c20) des Zahnersatzes und dreidimensionale Oberflächeninformationen (t12) auf einem passenden Zahn des passenden Zielabschnitts überlappt sind, als den überlappenden Okklusionsbereich (c31).

4. Verfahren zur Herstellung von Zahnersatz nach Anspruch 1, wobei in dem ersten Vorgang der Kopplungsbiss als Wachsbiss (310) zum Scannen bereitgestellt wird, und wobei der Wachsbiss zum Scannen umfasst:
einen variablen Formanpassungsabschnitt (311, 313, 315), der gebildet wird, während er entsprechend einem voreingestellten Standardzahnbogenprofil standardisiert wird und zwischen dem Implantationszielabschnitt (2) und dem passenden Zielabschnitt (3) entsprechend dem Implantationszielabschnitt angeordnet wird, während er erweicht wird, wenn er auf eine voreingestellte Temperatur oder höher erhitzt wird; und
einen vorstehenden Kauabschnitt (316), der einstückig mit dem variablen Formanpassungsabschnitt (311, 313, 315) ausgebildet ist und entsprechend dem passenden Zielabschnitt (3) vorsteht, während er erweicht wird, wenn er auf eine voreingestellte Temperatur oder höher erhitzt wird.

5. Verfahren nach Anspruch 4, wobei in dem ersten Vorgang der Wachsbiss (310) zum Scannen unter Druck gesetzt wird, so dass ein Spalt zwischen dem variablen Formanpassungsabschnitt (311, 313, 315) und dem vorstehenden Kauabschnitt (315), die innerhalb eines voreingestellten Temperaturbereichs erhitzt werden, um erweicht zu werden, der okklusalen vertikalen Dimension entspricht, während der variable Formanpassungsabschnitt (311, 313, 315), der einen Rand aufweist, der größer als eine Breite eines Standard-Gingivaabschnitts ist, korrigiert wird, um entsprechend mit einem äußeren Oberflächenprofil des Implantationszielabschnitts (2) formangepasst zu werden und integral mit dem formangepassten Korrekturabschnitt (311r) ausgebildet wird, und der vorstehende Kauabschnitt (315) korrigiert wird, um entsprechend mit dem passenden Zielabschnitt (3) zu okkludieren und integral mit einem zu installierenden okklusalen Korrekturabschnitt (312r) ausgebildet wird.

6. Verfahren zur Herstellung von Zahnersatz nach Anspruch 5, wobei in dem ersten Vorgang der formangepasste Korrekturabschnitt (311r) unterfüttert wird, so dass eine innere Oberfläche mit einem härtbaren Harz gefüllt und okkludiert wird, um einen Spalt zwischen dem formangepassten Korrekturabschnitt (311r) und dem Implantationszielabschnitt (2) auszugleichen.

7. Verfahren nach Anspruch 5, wobei bei das gescannte Bild in dem ersten Vorgang umfasst:
ein erstes gescanntes Bild (m11) der inneren und äußeren Oberflächenabschnitte des Wachsbisses (310) zum Scannen, das korrigiert wird, um den formangepassten Korrekturabschnitt (311r) und den okklusalen Korrekturabschnitt (312r) integral zu bilden;
ein zweites gescanntes Bild (m12) einer äußeren Oberfläche des passenden Zielabschnitts (3); und
ein drittes gescanntes Bild (m13) von äußeren Oberflächen des Implantationszielabschnitts (2) und des passenden Zielabschnitts (3), die zum Scannen mit dem Wachsbiss okkludiert sind.

8. Verfahren zur Herstellung von Zahnersatz nach Anspruch 7, wobei in dem ersten Vorgang eine Vielzahl solcher gescannter Bilder außerdem ein viertes gescanntes Bild der äußeren Oberfläche des Implantationszielabschnitts (3) umfasst,
wobei in dem ersten Vorgang das integrierte gescannte Bild (m17) durch Überlappen und Ausrichten des ersten gescannten Bildes (m11) mit dem zweiten gescannten Bild (m12) auf der Basis gemeinsamer Teile mit dem dritten gescannten Bild (m13), das der okklusalen vertikalen Dimension entspricht, erzeugt wird, während das erste gescannte Bild mit dem vierten gescannten Bild vertauscht wird, und
wobei in dem zweiten Vorgang das integrierte gescannte Bild (m17) durch Überlappen und Ausrichten des ersten gescannten Bildes (m11) mit dem zweiten gescannten Bild (m12) auf der Basis jedes gemeinsamen Teils mit dem dritten gescannten Bild (m13), das der okklusalen vertikalen Dimension entspricht, erzeugt wird, während das erste gescannte Bild (m11) ausgetauscht wird, um dreidimensionale Oberflächeninformationen des formangepassten Korrekturabschnitts (311r), der einem inneren Oberflächenabschnitt des Wachsbisses entspricht, zum Scannen äußerlich freizulegen.

9. Verfahren zur Herstellung eines Zahnersatzes, das umfasst:
einen ersten Vorgang (s510) zum Erzeugen eines dreidimensionalen Arbeitsbildes (1d), das Informationen über das Ziel und die passende Oberfläche enthält, durch Ausrichten und In-Übereinstimmung-Bringen eines gescannten Bildes (m17) und eines CT-Bildes (c16) eines Implantationszielabschnitts (2) und eines passenden Zielabschnitts (3) auf der Grundlage von Bildinformationen über eine okklusale vertikale Dimension für jede zu behandelnde Person;
einen zweiten Vorgang (s520) zum Erzeugen eines virtuellen provisorischen Zahnersatzes (14d), in dem innere und äußere Oberflächenabschnitte, die den Informationen über das Ziel und die passende Oberfläche entsprechen, in dem dreidimensionalen Arbeitsbild (1d) festgelegt sind, und zum Herstellen eines provisorischen Zahnersatzes (14) auf der Grundlage des virtuellen provisorischen Zahnersatzes (14d), wobei der provisorische Zahnersatz (14) unter Verwendung von Paraffinwachs hergestellt wird;
einen dritten Vorgang (s530) zum Erhalten eines Bewegungsbildes (c30) des gotischen Bogens einer dreidimensionalen Bewegungsspur eines Temporomandibulargelenks der zu behandelnden Person, während der provisorische Zahnersatz zwischen dem Implantationszielabschnitt (2) und dem passenden Zielabschnitt (3) installiert ist und ein Okklusionsflächenabschnitt (18) des provisorischen Zahnersatzes (14) mit dem passenden Zielabschnitt (3) okkludiert ist, und zum Erzeugens von Konstruktionsinformationen (c20) eines Zahnersatzes, der eine virtuelle Kaufläche enthält, auf der Grundlage eines dreidimensionalen Planungsbildes (1f), das durch Korrigieren der Informationen über das Ziel und die passende Oberfläche erhalten wird;
einen vierten Vorgang (s540) zum Korrigieren der Konstruktionsinformationen (c20) des Zahnersatzes durch Überlappen des Bewegungsbildes (c30) mit den Konstruktionsinformationen (c20) des Zahnersatzes, um einen überlappenden Okklusionsbereich (c31) auf der virtuellen Kaufläche anzuzeigen, und Setzen eines überlappenden Löschbereichs (c31r) in Bezug auf den überlappenden Okklusionsbereich (c31); und
einen fünften Vorgang (s550) zum Herstellen eines endgültigen Zahnersatzes durch Herstellen eines künstlichen Zahns, der den korrigierten Konstruktionsinformationen (c20a) des Zahnersatzes entspricht.

10. Verfahren zur Herstellung von Zahnersatz nach Anspruch 9, wobei der vierte Vorgang das Einstellen der virtuellen Kaufläche der korrigierten Konstruktionsinformationen (c20a) des Zahnersatzes umfasst, um wie eine gotische Bogenform vertieft zu sein, entsprechend der dreidimensionalen Bewegungsspur des Temporomandibulargelenks durch Löschen des überlappenden Löschbereichs (c31r), und
wobei der fünfte Vorgang das Herstellen einer Kaufläche des auf der Grundlage der korrigierten Konstruktionsinformationen (c20a) des Zahnersatzes hergestellten künstlichen Zahns umfasst, so dass sie wie die gotische Bogenform vertieft ist.

11. Verfahren zur Herstellung von Zahnersatz nach Anspruch 9, wobei der vierte Vorgang umfasst:
virtuelles Bewegen der Konstruktionsinformationen des Zahnersatzes, der virtuell auf dem dreidimensionalen Planungsbild (1f) angeordnet ist, entlang der dreidimensionalen Bewegungsspur auf der Basis des Bewegungsbildes unter Verwendung des Planungsabschnitts (330); und
Festlegen und Anzeigen, durch den Planungsabschnitt (330), eines Bereichs, in dem die Konstruktionsinformationen des Zahnersatzes und dreidimensionale Oberflächeninformationen auf einem virtuellen Okklusionsflächenabschnitt des passenden Zielabschnitts (3) überlappt sind, als den überlappenden Okklusionsbereich (c31).

12. Verfahren zur Herstellung von Zahnersatz nach Anspruch 9, wobei in dem dritten Vorgang das Bewegungsbild (c30) durch Bewegungsabtastung, unter Verwendung eines dynamischen Scanners, einer dreidimensionalen Bewegungsspur der Bewegung des Temporomandibulargelenks in einer Links-Rechts-Richtung, einer Vorne-Hinten-Richtung und einer vertikalen Richtung erhalten wird, während eine äußere Oberfläche der Mundhöhle freigelegt ist und der Okklusionsflächenabschnitt und der passende Zielabschnitt miteinander okkludiert sind.

13. Verfahren zur Herstellung von Zahnersatz nach Anspruch 9, wobei in dem zweiten Vorgang ein provisorischer korrigierter Oberflächenabschnitt (118a), der mit einer Querschnittsfläche vorsteht, die einem voreingestellten Standardzahnbogenprofil entspricht, auf dem provisorischen Zahnersatz (114) ausgebildet wird, während eine provisorische Friktionsmarkierung, die mindestens einen Kratzer oder ein Intaglio zur Verbesserung der Haftung mit einem härtbaren Harz, das sich während der Unterfütterung füllt, enthält, in einer Okklusionsfläche des provisorischen korrigierten Oberflächenabschnitts (118a) ausgebildet wird, und
wobei in dem dritten Vorgang die Informationen über das Ziel und die passende Oberfläche durch Füllen des provisorischen korrigierten Oberflächenabschnitts (118a) mit dem härtbaren Harz und Aushärten des härtbaren Harzes korrigiert werden, während das härtbare Harz gehärtet wird, während die okklusale Oberfläche des provisorischen korrigierten Oberflächenabschnitts (118a) aufgefüllt wird, so dass ein Spalt zwischen dem passenden Zielabschnitt (3) und dem provisorischen korrigierten Oberflächenabschnitt (118a) als ein provisorischer korrigierter vorstehender Abschnitt gebildet wird, der entsprechend der okklusalen vertikalen Dimension korrigiert wird.

14. Verfahren zur Herstellung von Zahnersatz nach Anspruch 9, wobei der erste Vorgang ferner das vorläufige Erhalten eines vorläufigen Bewegungsbildes des gotischen Bogens umfasst, während ein vorstehender Kauabschnitt, der in einem Wachsbiss (310) vorgesehen ist, welcher aus einem Paraffinwachsmaterial gebildet ist, das zwischen dem Implantationszielabschnitt (2) und dem passenden Zielabschnitt (3) angeordnet ist, und der passende Zielabschnitt miteinander okkludiert sind, und
wobei der zweite Vorgang ferner das vorläufige Korrigieren des virtuellen Zahnersatzes durch Anzeigen eines vorläufigen überlappenden Okklusionsbereichs auf einem virtuellen Okklusionsflächenabschnitt (r12) des virtuellen temporären Zahnersatzes durch Überlappen des vorläufigen Bewegungsbildes mit dem virtuellen temporären Zahnersatz (114d) und Setzen eines vorläufigen überlappenden Löschbereichs in Bezug auf den vorläufigen überlappenden Okklusionsbereich umfasst.

## Revendications

1. Un procédé de fabrication de restauration dentaire comprenant:
une première opération permettant d'obtenir à l'aide d'un dispositif de capture d'image (320), chacune d'une image numérisée d'une partie cible d'implantation (2) et d'une partie cible correspondante (3) avant l'installation d'une occlusion d'accouplement (309) disposée entre la partie cible d'implantation (2) et la partie cible correspondante (3) et après l'installation des parties de surface interne et externe de l'occlusion d'accouplement (309) par pression occlusale en tenant compte d'une dimension verticale occlusale d'une personne à traiter, une image tomodensitométrique (CT) (m16) d'une cavité buccale obstruée à l'aide de l'occlusion d'accouplement (309), et une image de mouvement d'arc gothique (c30) d'une trajectoire de mouvement tridimensionnelle d'une articulation temporo-mandibulaire de la personne à traiter alors qu'une partie saillante masticatoire prévue dans l'occlusion d'accouplement et la partie cible correspondante sont obstruées l'une par l'autre, et transmettre les images à une partie de planification (330);
une deuxième opération permettant de générer une image numérisée intégrée (m17) en alignant et en arrangeant une pluralité de ces images numérisées de manière à correspondre à la dimension verticale occlusale tout en générant une image de planification tridimensionnelle (m20) en superposant et en faisant correspondre les images numérisées sur la base des parties communes avec l'image CT (m16);
une troisième opération permettant de générer et de disposer des informations de conception (c20) d'une restauration dentaire comprenant une surface masticatoire virtuelle sur la base de l'image de planification tridimensionnelle (m20) et en superposant l'image de mouvement (c30) avec les informations de conception (c20) de la restauration dentaire de manière à afficher une région d'occlusion superposée (c31) sur la surface masticatoire virtuelle; et
une quatrième opération permettant de corriger des informations de conception (c20) de la restauration dentaire en définissant une région de suppression superposée (c31a) par rapport à la région d'occlusion superposée (c31) et transmettre les informations de conception corrigées (c20) de la restauration dentaire à un dispositif de fabrication (340) afin de fabriquer la restauration dentaire.

2. Le procédé de fabrication de restauration dentaire selon la revendication 1, dans lequel, lors de la première opération, l'image de mouvement (c30) est obtenue par balayage de mouvement, à l'aide d'un scanner dynamique, d'une trajectoire de mouvement tridimensionnelle du mouvement de l'articulation temporo-mandibulaire dans une direction gauche-droite, une direction avant-arrière et une direction verticale tandis qu'une surface externe de la cavité buccale est exposée et la partie saillante masticatoire et la partie cible correspondante sont obstruées l'une avec l'autre.

3. Le procédé de fabrication de restauration dentaire selon la revendication 1, dans lequel la troisième opération comprend:
déplacer virtuellement des informations de conception (c20) de la restauration dentaire qui sont virtuellement disposées sur l'image de planification tridimensionnelle (c20) le long de la trajectoire de mouvement tridimensionnelle sur la base de l'image de mouvement (c30) à l'aide de la partie de planification (330); et
définir et afficher, par la partie de planification (330), une zone dans laquelle les informations de conception (c20) de la restauration dentaire et les informations de surface tridimensionnelles (t12) sur une dent correspondante de la partie cible correspondante sont superposées en tant que région d'occlusion superposée (c31).

4. Le procédé de fabrication de restauration dentaire selon la revendication 1, dans lequel, dans la première opération, l'occlusion de couplage est (309) prévue sous forme de morsure de cire (310) pour numérisation, et dans lequel la morsure de cire pour numérisation comprend:
une partie de forme variable correspondante (311, 313, 315) formée tout en étant standardisée et correspondant à un profil d'arcade dentaire standard prédéfini et disposée entre la partie cible d'implantation (2) et la partie cible correspondante (3) correspondant à la partie cible d'implantation tout en étant ramollie lorsqu'elle est chauffée à une température prédéfinie ou supérieure; et
une partie saillante masticatoire (316) intégralement formée avec la partie de forme variable correspondante (311, 313, 315) et faisant saillie de manière à correspondant à la partie cible d'adaptation (3) tout en étant ramollie lorsqu'elle est chauffée à une température prédéfinie ou supérieure.

5. Le procédé selon la revendication 4, dans lequel, lors de la première opération, la morsure de cire (310) pour numérisation est mise sous pression de telle sorte qu'un espace entre la partie de forme variable correspondante (311, 313, 315) et la partie saillante masticatoire (315), qui sont chauffées dans une plage de température prédéfinie pour être ramollies, correspond à la dimension verticale occlusale tandis que la partie de forme variable correspondante (311, 313, 315) ayant une marge supérieure à la largeur d'une partie gingivale standard est corrigée pour correspondre en conséquence à la forme d'un profil de surface externe de la partie cible d'implantation (2) et intégralement formée avec une partie de correction de forme correspondante (311r), et la partie saillante masticatoire (315) est corrigée de manière à former une occlusion en conséquence avec la partie cible correspondante (3) et intégralement formée avec une partie de correction occlusale (312r) à installer.

6. Le procédé de fabrication de restauration dentaire selon la revendication 5, dans lequel, lors de la première opération, la partie de correction de forme correspondante (311r) est regarnie de sorte qu'une surface interne est remplie et obstruée par une résine durcissable pour compenser un espace entre la partie de correction de forme correspondante (311r) et la partie cible d'implantation (2).

7. Le procédé selon la revendication 5, dans lequel, lors de la première opération, l'image numérisée comprend:
une première image numérisée (m11) des parties de surface interne et externe de la morsure de cire (310) pour numérisation qui est corrigée pour former intégralement la partie de correction de forme correspondante (311r) et la partie de correction occlusale (312r);
une deuxième image numérisée (m12) d'une surface externe de la partie cible correspondante (3); et
une troisième image numérisée (m13) des surfaces externes de la partie cible d'implantation (2) et de la partie cible correspondante (3) qui sont obstruées par la morsure de cire pour numérisation.

8. Le procédé de fabrication de restauration dentaire selon la revendication 7, dans lequel, lors de la première opération, une pluralité de ces images numérisées comprend en outre une quatrième image numérisée de la surface externe de la partie cible d'implantation (3),
dans lequel lors de la première opération, l'image numérisée intégrée (m17) est générée en superposant et en alignant la première image numérisée (m11) avec la deuxième image numérisée (m12) sur la base des parties communes avec la troisième image numérisée (m13) correspondant à la dimension verticale occlusale tout en permutant la première image numérisée avec la quatrième image numérisée, et
dans lequel lors de la deuxième opération, l'image numérisée intégrée (m17) est générée en superposant et en alignant la première image numérisée (m11) avec la deuxième image numérisée (m12) sur la base des parties communes avec la troisième image numérisée (m13) correspondant à la dimension verticale occlusale tout en permutant la première image numérisée (m11) pour exposer extérieurement les informations de surface tridimensionnelles de la partie de correction de forme correspondante (311r) pour correspondre à une partie de surface interne de la morsure de cire (310) pour numérisation.

9. Un procédé de fabrication de restauration dentaire comprenant:
une première opération (s510) permettant de générer une image de travail tridimensionnelle (1d) comprenant des informations de cible et de surface d'adaptation en alignant et en faisant correspondre une image numérisée (m17) et une image CT (c16) d'une partie cible d'implantation (2) et d'une partie cible correspondante (3) sur la base d'informations d'image sur une dimension verticale occlusale pour chaque personne à traiter;
une deuxième opération (s520) permettant de générer une restauration dentaire temporaire virtuelle (14d) dans laquelle des parties de surface interne et externe correspondant aux informations de cible et de surface correspondantes sont définies dans l'image de travail tridimensionnelle (1d) et fabriquer une restauration dentaire temporaire (14) sur la base de la restauration dentaire temporaire virtuelle (14d), dans laquelle ladite restauration dentaire temporaire (14) est fabriquée à l'aide de cire paraffinique;
une troisième opération (s530) permettant d'obtenir une image de mouvement d'arcade gothique (c30) d'une trajectoire de mouvement tridimensionnelle d'une articulation temporo-mandibulaire de la personne à traiter pendant que la restauration dentaire temporaire est installée entre la partie cible d'implantation (2) et la partie cible correspondante (3) et une partie de surface occlusale (18) de la restauration dentaire temporaire (14) est obstruée par la partie cible correspondante (3) et génère des informations de conception (c20) d'une restauration dentaire comprenant une surface masticatoire virtuelle sur la base d'une image de planification tridimensionnelle (lf) obtenue en corrigeant la cible et en faisant correspondre les informations de surface;
une quatrième opération (s540) permettant de corriger les informations de conception (c20) de la restauration dentaire en superposant l'image de mouvement (c30) avec les informations de conception (c20) de la restauration dentaire pour afficher une région d'occlusion superposée (c31) sur la surface masticatoire virtuelle et définir une région de suppression superposée (c31r) par rapport à la région d'occlusion superposée (c31); et
une cinquième opération (s550) permettant de fabriquer une restauration dentaire finale en fabriquant une dent artificielle correspondant aux informations de conception corrigées (s20a) de la restauration dentaire.

10. Le procédé de fabrication de restauration dentaire selon la revendication 9, dans lequel la quatrième opération comprend la définition de la surface masticatoire virtuelle des informations de conception corrigée (c20a) de la restauration dentaire de manière à être en_retrait pour avoir une forme d'arcade gothique correspondant à la trajectoire de mouvement tridimensionnelle de l'articulation temporo-mandibulaire en supprimant la région de suppression superposée (c31r), et
dans lequel la cinquième opération permet de fabriquer une surface masticatoire de la dent artificielle fabriquée sur la base des informations de conception corrigées (c20a) de la restauration dentaire de manière à être en retrait comme la forme de l'arcade gothique.

11. Le procédé de fabrication de restauration dentaire selon la revendication 9, dans lequel la quatrième opération comprend:
déplacer virtuellement les informations de conception de la restauration dentaire qui sont disposées virtuellement sur l'image de planification tridimensionnelle (1f) le long de la trajectoire de mouvement tridimensionnelle sur la base de l'image de mouvement en utilisant la partie de planification (330); et
définir et afficher, par la partie de planification (330), une zone dans laquelle les informations de conception de la restauration dentaire et les informations de surface tridimensionnelles sur une partie de surface occlusale virtuelle de la partie cible correspondante (3) sont superposées en tant que région d'occlusion superposée (c31).

12. Le procédé de fabrication de restauration dentaire selon la revendication 9, dans lequel lors de la troisième opération, l'image de mouvement (c30) est obtenue par balayage de mouvement, à l'aide d'un scanner dynamique, d'une trajectoire de mouvement tridimensionnelle du mouvement de l'articulation temporo-mandibulaire dans une direction gauche-droite, avant-arrière et verticale tandis qu'une surface externe de la cavité buccale est exposée et la partie de la surface occlusale et la partie cible d'adaptation sont obstruées l'une par l'autre.

13. Le procédé de fabrication de restauration dentaire selon la revendication 9, dans lequel, lors de la deuxième opération, une partie de surface corrigée temporaire (118a) dépassant d'une section transversale correspondant à un profil d'arcade dentaire standard prédéfini est formée sur la restauration dentaire temporaire (114) tandis qu'un marqueur de friction temporaire comprenant au moins une rayure et une intaille pour améliorer l'adhérence avec une résine durcissable qui est remplie pendant le regarnissage est formé dans une surface occlusale de la partie de surface corrigée (118a), et
dans lequel lors de la troisième opération, les informations de cible et de surface correspondantes sont corrigées en remplissant la partie de surface temporaire corrigée (118a) avec la résine durcissable et en procédant au durcissement de la résine durcissable tout en remplissant la surface occlusale de la partie de surface temporaire corrigée (118a) de sorte qu'un espace entre la partie cible correspondante (3) et la partie de surface temporaire corrigée (118a) est formé en tant que une partie saillante temporaire corrigée correspondant à la dimension verticale occlusale.

14. Le procédé de fabrication de restauration dentaire selon la revendication 9, dans lequel la première opération comprend en outre l'obtention préliminaire d'une image de mouvement d'arcade gothique tandis qu'une partie saillante masticatoire prévue dans une morsure de cire (310) formée d'un matériau de cire paraffinique disposée au milieu la partie cible d'implantation (2) et la partie cible correspondante (3), et la partie cible correspondante étant obstruée par l'autre, et
dans lequel la deuxième opération comprend en outre la correction préliminaire de la restauration dentaire virtuelle en affichant une région d'occlusion préliminaire superposée sur une partie de surface occlusale virtuelle (r12) de la restauration dentaire temporaire virtuelle en superposant l'image de mouvement préliminaire avec la restauration dentaire temporaire virtuelle (114d) et en définissant une région de suppression préliminaire superposée par rapport à la région d'occlusion préliminaire superposée.
